(19) Europäisches Patentamt
European Patent Office
Office européen des brevets

(11) EP 4 299 131 A1

(12) EUROPEAN PATENT APPLICATION

(43) Date of publication:
**03.01.2024 Bulletin 2024/01**

(21) Application number: **22305943.7**

(22) Date of filing: **29.06.2022**

(51) International Patent Classification (IPC):
***A61P 31/00*** $^{(2006.01)}$ ***A61L 2/08*** $^{(2006.01)}$
***C09B 57/00*** $^{(2006.01)}$

(52) Cooperative Patent Classification (CPC):
**A61P 31/00; A61L 2/088; C09B 57/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Centre national de la recherche scientifique**
  **75016 Paris (FR)**
- **Nantes Université**
  **44000 Nantes (FR)**
- **University of Chile**
  **7500000 SANTIAGO (CL)**
- **Université de Limoges**
  **87000 Limoges (FR)**

(72) Inventors:
- **COEFFARD, Vincent**
  **44140 Le Bignon (FR)**
- **DE BONFILS, Paul**
  **44000 Nantes (FR)**
- **NUN, Pierrick**
  **44100 Nantes (FR)**
- **ZITOUNI, Béchir**
  **44620 La Montagne (FR)**
- **SOL, Vincent**
  **87000 Limoges (FR)**
- **VERNISSE, Charlotte**
  **03220 Trezelles (FR)**
- **OUK, Tan-Sothea**
  **87620 Sereilhac (FR)**
- **GUNTHER, German**
  **Santiago de Chile (CL)**

(74) Representative: **Novagraaf Technologies**
**Bâtiment O2**
**2, rue Sarah Bernhardt**
**CS90017**
**92665 Asnières-sur-Seine Cedex (FR)**

# (54) HYDROPHILIC PHENALENONES AND THEIR USE IN ANTIMICROBIAL PHOTODYNAMIC THERAPY

(57) The present invention belongs to the field of antimicrobial compositions and antimicrobial photodynamic therapy, and more specifically to hydrophilic phenalenones and their use in said field. It relates more specifically to new yellow-fluorescent phenalenone photosensitizers for applications in antimicrobial photodynamic therapy (aPDT).

The present invention relates to new hydrophilic phenalenones compounds and their use as a medicine, in antimicrobial photodynamic therapy, and more specifically in the treatment of various diseases, such as acne vulgaris, periodontis infections, rosacea, ptyriasis versicolor, molluscum contagiosum, genital herpes, tinea pedis, endodontics infections, viral keratitis, COVID-19 or influenza. The invention also relates to the method of synthesis of new hydrophilic phenalenone compounds according to the invention.

EP 4 299 131 A1

## Description

### Technical field

The present invention belongs to the field of antimicrobial compositions and antimicrobial photodynamic therapy, and more specifically to hydrophilic phenalenones and their use in said field. It relates more specifically to new yellow-fluorescent phenalenone photosensitizers for applications in antimicrobial photodynamic therapy (aPDT).

The present invention relates to new hydrophilic phenalenones compounds and their use as a medicine, in antimicrobial photodynamic therapy, and more specifically in the treatment of various diseases, such as acne vulgaris, periodontis infections, rosacea, ptyriasis versicolor, molluscum contagiosum, genital herpes, tinea pedis, endodontics infections, viral keratitis, COVID-19 or influenza. The invention also relates to the method of synthesis of new hydrophilic phenalenone compounds according to the invention.

In the description below, references between **[]** refer to the list of references at the end of the examples.

### Technical background

Antimicrobial resistance is a major worldwide threat that requires effective actions and innovative long-term solutions from researchers, policy-makers and society as a whole **[1,2].** In a report published in 2019, the Interagency Coordination Group on Antimicrobial Resistance estimated that at least 700,000 deaths globally a year involved drug resistance with gloomy predictions in the decades to come if no actions are taken **[3].**

Antibiotic misuse and overuse for human and animal applications have accelerated the emergence of virulent and antibiotic-resistant pathogens which are difficult to eradicate with the current medicines **[4,5].** The discovery of efficient and long-term viable therapeutic solutions, particularly against drug-resistant bacterial pathogens, is urgently required to tackle the challenge of antimicrobial resistance.

Amongst the possible strategies, antimicrobial photodynamic therapy (aPDT) has many advantages to foster innovation in the development of new therapeutic solutions **[6-8].** Antimicrobial photodynamic therapy involves three key components for killing bacterial pathogens: a photosensitizer, a light source and oxygen **[9].** Upon irradiation, the triplet excited state of the photosensitizer is populated from singlet excited state *via* intersystem crossing (ISC) **[10,11].** In type II photosensitized oxidations, the triplet excited state of the photosensitizer is quenched by ground-state oxygen leading to the formation of singlet oxygen, $^1O_2$, *via* energy transfer while type I photoreactions is based on electron transfer processes with the excited photosensitizer leading to radical species involved in the oxidative processes **[12].** The biocidal reactive oxygen species formed during photodynamic therapy can react with a variety of biomolecules such as nucleic acids, lipids and proteins leading to the death of pathogens **[13,14].**

This non-selective mode of action makes aPDT less keen to induce resistance and this is an attractive feature in the context of antibiotic resistance **[15].** The design of photoantimicrobial agents has attracted considerable attention from the scientific community over the past decades **[16].**

However, for applications in antimicrobial photodynamic therapy, the photosensitizer has to fulfill some criteria:

(i) strong absorption of visible light with efficient production of reactive oxygen species;
(ii) high photostability under aPDT conditions;
(iii) non-toxicity to the human host and high selectivity towards microbes;
(iv) appropriate water solubility; and
(v) cost-effective and straightforward synthetic processes to prepare the photosensitizer **[7,17].**

Based on these considerations, phenalenone is an attractive scaffold to build type II photoantimicrobial agents owing to a high quantum yield of intersystem crossing, high energy transfer efficiency from the triplet excited state of phenalenone to form singlet oxygen from ground-state oxygen and its photostability **[18-21].** As a result, the combination of the phenalenone motif with cationic substituents such as ammonium or pyridinium **[22-28]** and phosphonium **[29]** has led to the emergence of hydrophilic photosensitizers with exciting antibacterial activities. The introduction of the cationic substituents plays a dual role by increasing the solubility in aqueous media and by improving the interaction of the photosensitizer with the negatively charged bacteria cell wall **[30].**

For example, the patent application WO2012/113860 **[43]** discloses novel photosensitizers which inactivate microorganisms more efficiently with a compound having the formula (a):

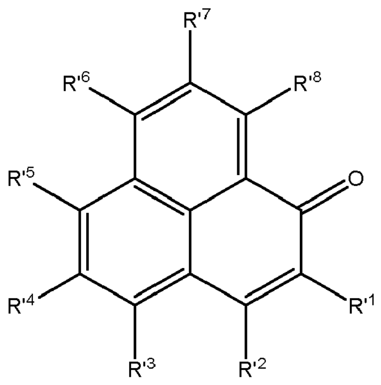

Formula (a)

where R'1 through R'8, which can be identical or different from each other, each are selected from of the group comprising hydrogen and the radical - $(CH_2)_k$-X, where k is a whole number from 1 to 20 and where X is an organic radical comprising a) at least one neutral, protonizable nitrogen atom and/or b) at least one positively charged nitrogen atom, under the provision that at least 1 radical R'1 through R'8 is not hydrogen, and one of these compounds is know in the field as SAPYR **[27-28].**

For example, the patent application WO2018/167264 **[44]** relates to a photosensitizer composition comprising one phenalene-1-one of formula (a), which ensures easy application to preferably different types of surfaces and, in particular, at the same time exhibits good antimicrobial activity after exposure to electromagnetic radiation of suitable wavelength and intensity. The exemplified compounds comprise only one substituent, either on position R'1 or R'2 in formula (a).

Despite great accomplishments in the search for phenalenone photoantimicrobials, some challenges remain to be addressed. For instance, synthetic strategies towards functionalized phenalenone photoantimicrobials remain limited and most of the photosensitizers absorb in UVA regions which lowers their interest for medical purposes **[31].**

In addition, the development of phenalenone photosensitizers exhibiting a proper balance between fluorescence and intersystem crossing rates have not been reported for antibacterial applications **[32].** New compounds with such molecular architectures could pave the way to theranostic agents which would have a therapeutic action (singlet oxygen production) and would allow a diagnostic by fluorescence imaging.

**Detailed description of the invention**

Applicant has developed new hydrophilic phenalenones compounds that solve all of the problems listed above.

To address the limitations associated with known phenalenone-based photosensitizers such as those described in WO2012/113860 or WO2018/167264 **[43,44],** the Applicants surprisingly found out the compound object of the present invention is particularly efficient in antimicrobial therapeutic and non-therapeutic application, while in the meantime, it does not have the problems associated to known phenalenone derivatives.

The present invention relates to new hydrophilic phenalenone compounds, their synthetic methods and their applications, such as their use as a medicine or to clean surfaces from fungi, bacteria, viruses, parasites or chemical compounds.

The hydrophilic phenalenone compounds of the invention present the advantage to be easily synthesised, in mild conditions and present an antimicrobial activity. They also have the following advantages:

- they can be used as blue-absorbing dyes;
- they possess a modularity of substituents borne by the phenalenone framework which allows blue-absorption, non-aggregation properties and introduction of cationic substituents at 8-position on the phenalenone skeleton;
- they can be used as yellow-fluorescent theranostic agents;
- they are photostable; and
- they possess antibacterial activities under light.

The main features of the photosensitizers include a quaternary ammonium or heterocyclic onium (e.g pyridinium) unit for increasing the hydrophilicity and for improving the interaction with the bacteria cell wall, and an alkoxy group enabling blue light absorption. In some applications, the Ar group helps preventing aggregation between the photosensitizers.

Photophysical measurements have demonstrated that the photosensitizers produce singlet oxygen upon irradiation under blue light and exhibit yellow fluorescence.

Antibacterial photodynamic inactivation studies revealed promising phototoxicity activities of the photosensitizers against Gram-positive (*Staphylococcus aureus*) and Gram-negative bacteria (*Pseudomonas aeruginosa*).

The invention thus relates in a first aspect to a compound of formula I:

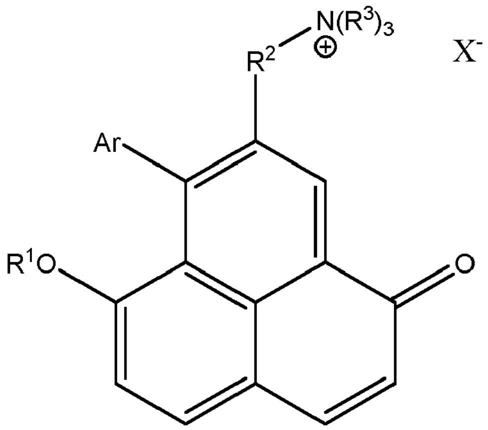

Formula I

wherein,

- $R^1$ represents a $C_1$ to $C_{12}$ linear or branched, saturated or unsaturated, hydrocarbon chain, optionally bearing a substituted or unsubstituted 4- to 26-membered aryl or heteroaryl moiety,
- Ar represents a substituted or unsubstituted 4- to 30-membered aryl or heteroaryl moiety,
- $R^2$ represents a $C_1$ to $C_3$ linear or branched alkyl chain,
- $X^-$ represents an anion,
- $R^3$ which independently of one another may be identical to or different from each other, respectively represent:
  - a $C_1$ to $C_{10}$ linear or branched alkyl chain, optionally bearing a substituted or unsubstituted, saturated or unsaturated, aromatic or non-aromatic, 4- to 26-membered cyclic or heterocyclic moiety,
  - a $C_1$ to $C_{26}$ linear or branched hydroxyalkyl group, and/or
  - at least two $R^3$ groups form, together with the nitrogen atom, a substituted or unsubstituted, saturated or unsaturated, aromatic or non-aromatic, 4- to 10-membered heterocyclic moiety.

The compounds of formula I comprise a phenalenone ring. As meant herein, a phenalene-1-one ring is of formula II:

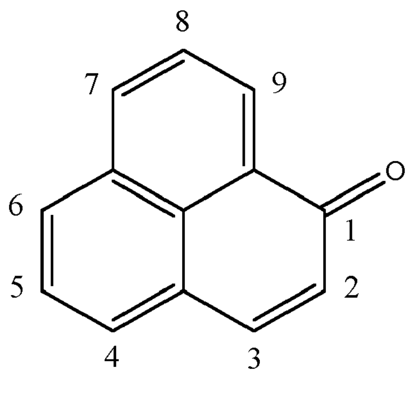

Formula II

The compounds according to the invention are phenalenones derivatives where the position 6 has been grafted with $-OR^1$, the position 7 by Ar and the position 8 by $-R^2[N(R^3)_3]^+$.

An important feature of photosensitizers according to the invention is the presence of an alkoxy group (preferably methoxy) on the phenalenone framework leading to UV-Vis. absorption in the blue region **[31]** which is suitable for the treatment of accessible infections (i.e. skin, mouth, eyes) **[33]** or the eradication of bacterial biofilms on materials. Another important feature is the presence of the Ar group (preferably phenyl) on the phenalenone framework leading to an additional diversity site. Finally, compared to the known compounds, the ammonium or pyridinium group is not on the

position 2, which allows to [increase the hydrophilicity and the ability of the phenanelone to interact with the bacteria cell wall. Surprisingly, the combination of these three groups, on three particular positions on the phenalenone rings could not have been anticipated by the person skilled in the art.

It is meant by "aryl", a group derived from arenes by removal of a hydrogen atom from a ring carbon atom; arenes being monoyclic and polycyclic aromatic hydrocarbons (IUPAC). According to the invention Ar may comprise from 4 to 30 carbon atoms, preferably 4 to 15 carbon atoms, more preferably 6 carbon atoms (e.g. phenyl group). Heteroaryl are the class of heterocyclic groups derived from heteroarenes by removal of a hydrogen atom from any ring atom; an alternative term is hetaryl. Heteroarenes being heterocyclic compounds formally derived from arenes by replacement of one or more methine (-C=) and/or vinylene (-CH=CH-) groups by trivalent or divalent heteroatoms, respectively, in such a way as to maintain the continuous $\pi$-electron system characteristic of aromatic systems and a number of out-of-plane $\pi$-electrons corresponding to the Hückel rule (4 n+2) (IUPAC).

Advantageously, $R^1$ may represent a $C_1$ to $C_{12}$ linear or branched, saturated or unsaturated, hydrocarbon chain, optionally bearing a substituted or unsubstituted 4- to 26-membered aryl or heteroaryl moiety. Preferably, $R^1$ may represent a $C_1$ to $C_6$ linear or branched, saturated or unsaturated, hydrocarbon chain, optionally bearing a substituted or unsubstituted 4- to 10-membered aryl or heteroaryl moiety. The aryl or heteroaryl moiety may be substituted by $-OR^4$, in which $R^4$ represents a $C_1$ to $C_3$ alkyl chain, preferably Me. More preferably, $R^1$ may be chosen from a $C_1$ to $C_4$ saturated or unsaturated, hydrocarbon chain, a benzyl group (i.e $C_1$ hydrocarbon chain bearing a phenyl group) and a methoxybenzyl group (i.e $C_1$ hydrocarbon chain bearing a methoxy substituted phenyl group). For example, $R^1$ may represent a group chosen from methyl, ethyl, isopropyl, n-propyl, n-butyl, n-propenyl, n-propynyl, benzyl and methoxybenzyl.

Advantageously, $R^1$ may be chosen from the following groups:

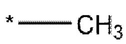 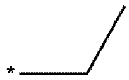 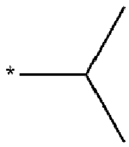 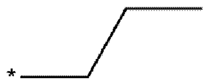 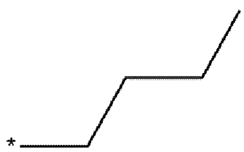

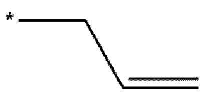 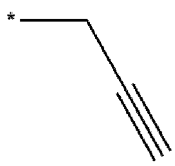 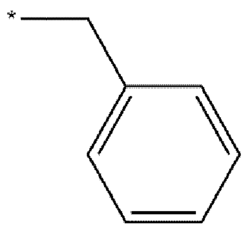 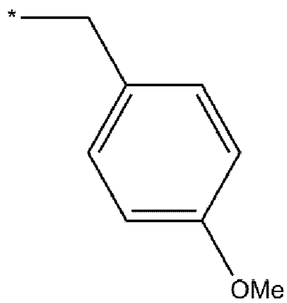

wherein * represents the point of attachment to the phenalenone ring. Preferably, $R^1$ may be chosen from the following groups:

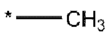 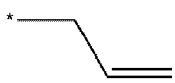 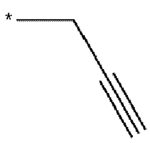 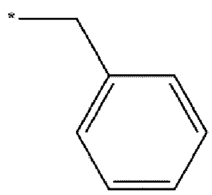 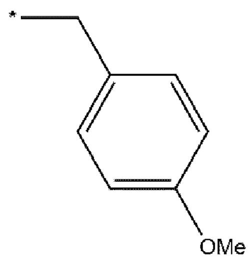

wherein * represents the point of attachment to the phenalenone ring.

Advantageously, Ar represents a substituted or unsubstituted 4- to 30-membered aryl or heteroaryl moiety, preferably a substituted or unsubstituted 4- to 15-membered aryl or heteroaryl moiety. The aryl or heteroaryl moiety may be substituted by one or two, identical or different, groups chosen from halo (e.g. Cl, Br, I), $-N_3$, $-OR^4$, $-NO_2$, $-R^4$, -CN,

-CHO, - $COR^4$, $-CO_2R^4$, phenyl, $2-R^4C_6H_4$ and 2-pyridyl, in which $R^4$ represents a $C_1$ to $C_3$ alkyl chain, preferably Me. Preferably, Ar is chosen from a substituted or unsubstituted 4- to 15-membered aryl or heteroaryl moiety. Ar may represent a group chosen from substituted or unsubstituted phenyl, naphtyl and anthracyl group.

Advantageously, Ar may be chosen from the following groups:

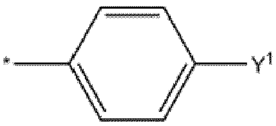
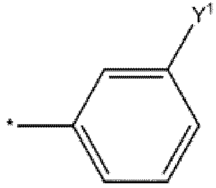
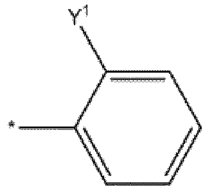

$Y^1 = H, Cl, Br, I, N_3, OR^4, NO_2, Me, CN, CHO, COMe, CO_2R^4$

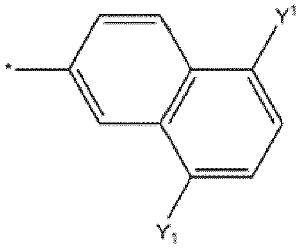
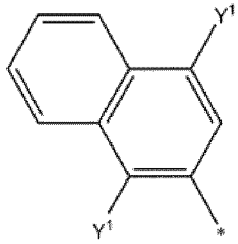
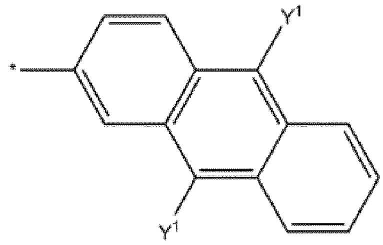

$Y^1 = H, Me, Ph, 2-MeC_6H_4$, 2-pyridyl

wherein * represents the point of attachment to the phenalenone ring. Preferably, Ar may be chosen from the following groups:

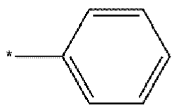
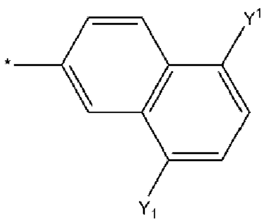
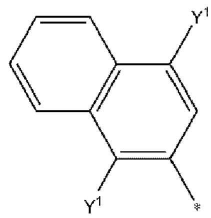
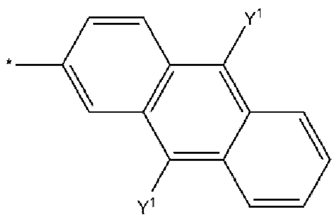

$Y^1 = H, Me, Ph, 2-MeC_6H_4$, 2-pyridyl

wherein * represents the point of attachment to the phenalenone ring.

Advantageously, $R^2$ represents a $C_1$ to $C_3$ linear or branched alkyl chain. Preferably, $R^2$ is $-CH_2-$.

Advantageously, $R^3$ which independently of one another may be identical to or different from each other, respectively represent:

- a $C_1$ to $C_{10}$ linear or branched alkyl chain, optionally bearing a substituted or unsubstituted, saturated or unsaturated, aromatic or non-aromatic, 4- to 26-membered cyclic or heterocyclic moiety,
- a $C_1$ to $C_{26}$ linear or branched hydroxyalkyl group, and/or
- at least two $R^3$ groups form, together with the nitrogen atom, a substituted or unsubstituted, saturated or unsaturated, aromatic or non-aromatic, 4- to 10-membered heterocyclic moiety.

Preferably, the substituted or unsubstituted, saturated or unsaturated, aromatic or non-aromatic, 4- to 26-membered cyclic or heterocyclic moiety may be substituted by one or two, identical or different groups, chosen from halo (e.g. Br), $-N_3$, $-NO_2$, -CN, $-OR^4$, $-R^4$, phenyl, $2-R^4C_6H_4$ and 2-pyridyl, in which $R^4$ represents a $C_1$ to $C_3$ alkyl chain, preferably Me. The $R^3$ groups may be independently chosen from methyl, hydroxyethyl, $C_1$ to $C_4$ N-alkylpyridin-2(1H)-one, substituted or unsubstituted benzyl group (i.e. $C_1$ alkyl chain bearing a substituted or unsubstituted phenyl group), substituted or unsubstituted naphthalen-2-ylmethylgroup (i.e. $C_1$ alkyl chain bearing a substituted or unsubstituted naphtyl group) and substituted or unsubstituted anthracen-2-ylmethyl group (i.e. $C_1$ alkyl chain bearing a substituted or unsubstituted anthracyl group) or the $R^3$ groups may form, together with the nitrogen atom, a substituted or unsubstituted heteroaryl group, preferably a pyridinyl group.

Advantageously, $R^3$ may independently be chosen from the following groups:

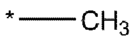 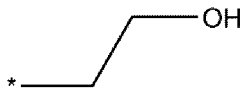 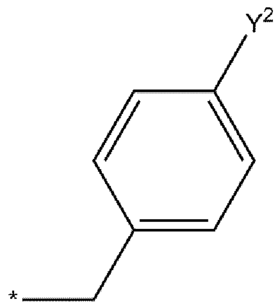 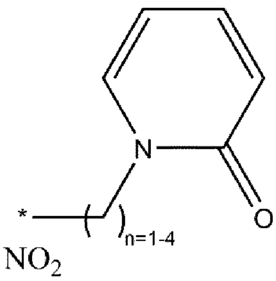

$Y^2$ = H, $OR^4$, Br, CN, $N_3$, $NO_2$

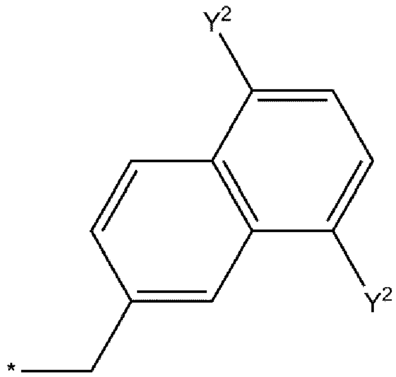 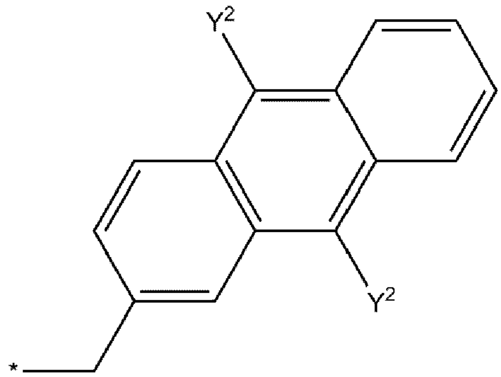

$Y^2$ = H, Me, Ph, 2-$MeC_6H_4$, 2-Pyridyl

wherein * represents the point of attachment to the nitrogen atom. Preferably, $R^3$ may be chosen from the following groups:

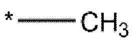 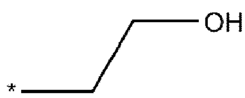 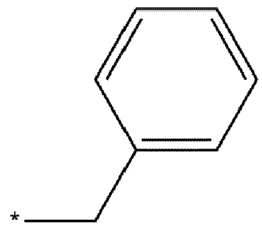

wherein * represents the point of attachment to the nitrogen atom.

Advantageously, the $R^3$ groups may form, together with the nitrogen atom, a substituted or unsubstituted pyridinyl group that may be represented as follow:

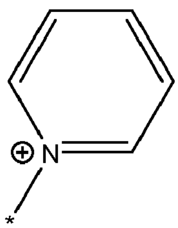

wherein * represents the point of attachment to the phenalenone ring.

Advantageously, $X^-$ represents an anion. X- may be chosen from $Cl^-$, $Br^-$, $I^-$, $PF_6^-$, $BF_4^-$, $TsO^-$.

Advantageously, the compounds according to the invention may be chosen from the following compounds:

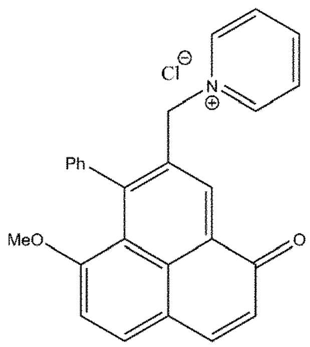

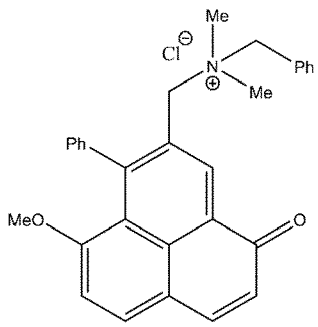

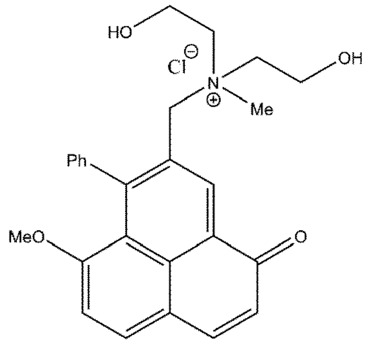

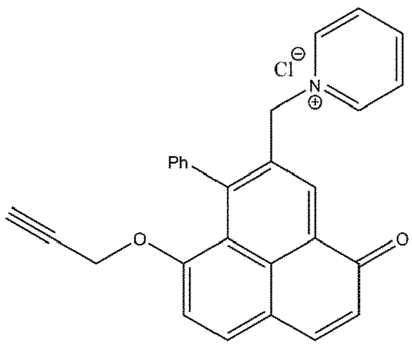

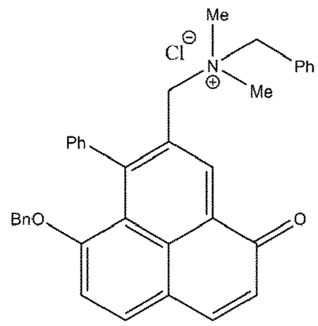

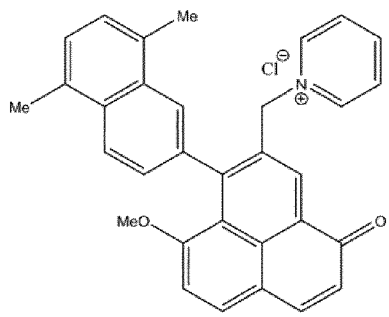

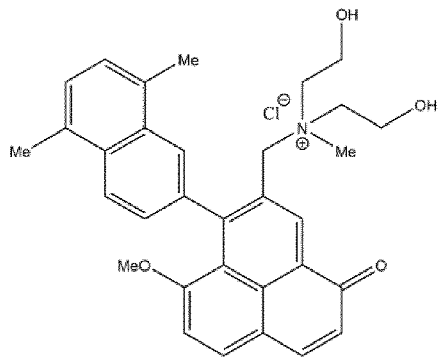

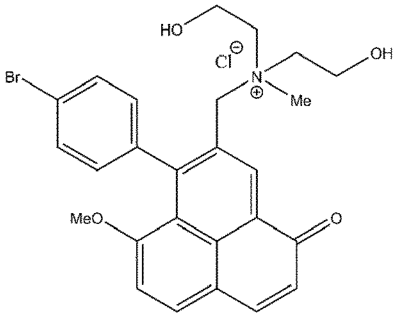

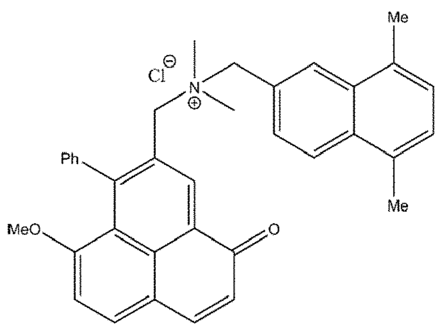

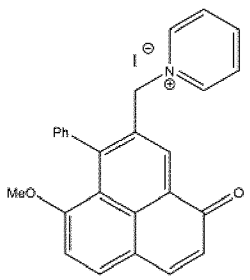

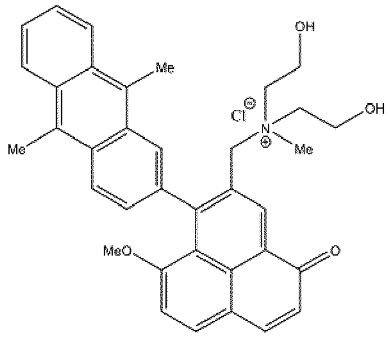

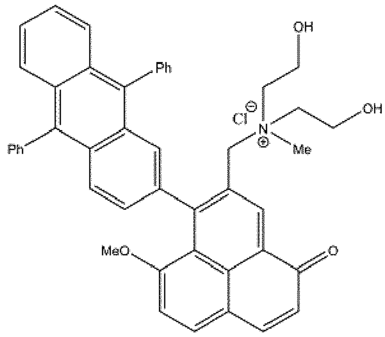

Advantageously, for each of the above listed compounds, the anion $Cl^-$ or $I^-$ may be different, and chosen from $Cl^-$, $Br^-$, $I^-$, $PF_6^-$, $BF_4^-$ and $TsO^-$.

The invention also relates to a pharmaceutical composition comprising a compound according to any of preceding claims, a solvent, preferably water, and optionally at least one pharmaceutically acceptable excipient.

Advantageously, the solvent may be water, and/or an organic solvent chosen from DMSO, MeOH, EtOH, MeCN and DMF.

Advantageously, the pharmaceutically acceptable excipient may be chosen from:

- polyols (e.g. propylene-glycol or glycerol),
- oils (from plant origin),
- waxes (e.g white wax, lanolein or carnauba wax),
- mineral oils (e.g. hydrocarbon based oil, vaseline or paraffine) and silicone oils,
- sugars and hydrophilic polymers (e.g saccharose, lactose, sorbitol, starch, gums such as arabic and tragacanth, alginates, cellulose such as cellulose and methylcellulose),
- proteins such as gelatin,
- synthetic polymers (e.g. polyvinylpyrrolidone or P.E.G.),
- mineral compounds (e.g. silica, talc, titanium oxide and various silicates such as kaolin, bentonites),
- anionic, cationic and/or non-ionic surfactants, and
- liposomes.

The invention also relates to compound or composition according to the invention, for use as a medicine. The medicine may be an antimicrobial agent or a theranostic agent.

The invention also relates to compound or composition according to the invention, for use in antimicrobial photodynamic therapy. It is meant herein by "antimicrobial photodynamic therapy" (aPDT), a method that involves the use of light with appropriate wavelength to kill microorganisms treated with a photosensitizer drug. aPDT could be a useful adjunct to mechanical as well as antibiotics in eliminating periopathogenic bacteria.

Advantageously, the compound or composition according to the invention may be for use in the treatment of various diseases or infections, such as for example acne vulgaris, periodontis infections, rosacea, ptyriasis versicolor, molluscum contagiosum, genital herpes, tinea pedis, endodontics infections, viral keratitis, COVID-19 or influenza. This list should not be considered limitative and various other diseases or infections may be treated with the compound or composition according to the invention.

Advantageously, the compound or composition according to the invention may be for use in odontology. For example, it may be useful in the treatment of oral candidiasis, tooth decay and periodontitis.

On another aspect, the invention also relates to an antimicrobial composition comprising a compound according to any of preceding claims 1-5, a solvent, preferably water or an organic solvent chosen from MeOH, EtOH, DMSO, DMF and MeCN and optionally at least one excipient.

The invention also encompasses a use of a compound or composition according to the invention, as an antimicrobial agent. The compound or composition according to the invention may be used for the eradication of microbial biofilms from surfaces. It may preferably be used for the treatment of surfaces. The surfaces may be surfaces of medical tools or any apparatus/tool/device found in a medical environment. The compound or composition according to the invention may also be used in paint for building or any surfaces that requires the elimination of microorganisms to be safely used. The compound or composition according to the invention may thus also be used for application for application of antimicrobial photodynamic therapy in the food industry. For instance, the compound according to the invention may be used for inactivation of pathogens on food surface, eradication of biofilms on equipment surfaces (e.g. conveyor belts, pipes) and packaging materials (e.g. glass, polystyrene). The invention thus also encompasses use of the compound or composition according to the invention in non-therapeutic applications of antimicrobial photodynamic therapy.

**Brief description of the figures:**

Figure 1 represents the UV/vis absorption spectra of **1a-c** measured in water solutions.

Figure 2 represents the emission spectra of compounds **1a** (A), **1b** (B) and **1c** (C) in different solvents (PS **1a-c** were excited at 400 nm).

Figure 3 represents the multi-step synthetic sequence towards hydrophilic phenalenones **1a-c.**

## EXAMPLES

### Example 1: Preparation of 1a, 1b and 1c according to the invention

**General *information:*** $^1$H NMR and $^{13}$C spectra were recorded at room temperature on samples dissolved in deuterated solvents. Chemical shifts (δ) are given in parts per million and coupling constants are given as absolute values expressed in Hertz. Structural assignments were made with additional information from COSY, HSQC and HMBC experiments. Electrospray (ES)-time of flight (TOF) mass spectrometry (MS) measurements were performed on a Xevo G2-XS QTOF spectrometer (Waters, USA) for ES+, ES-. An Atmospheric Solid Analysis Probe (ASAP) was used for the direct analysis of samples by atmospheric pressure ionization (ASAP+ or ASAP-). Calculated masses for the molecules are the sum of the atomic masses and do not consider the gain or loss of one electron. FTIR spectra were obtained from neat samples using the attenuated total reflection (ATR) technique. Thin-layer chromatography (TLC) was carried out on aluminum sheets precoated with silica gel. Column chromatography separations were performed using silica gel. All reagents were used without purification. All solvents were of HPLC grade or were distilled using standard drying agents prior to use. Starting chemical substrates and reagents were used as commercially provided unless otherwise indicated. SAPYR was prepared according to literature data **[27].** Compound **2** was prepared according to literature data **[31].**

**Experimental procedures:**

8-(hydroxymethyl)-6-methoxy-7-phenyl-1*H*-phenalen-1-one **3**

6-methoxy-1-oxo-7-phenyl-1*H*-phenalene-8-carbaldehyde **2** (100 mg, 0.32 mmol, 1 equiv.) was introduced in absolute ethanol (3.5 mL) and sodium borohydride (13.23 mg, 0.36 mmol, 1.1 equiv.) was then added. The reaction was left to stir for 2 hours 30 minutes. The reaction mixture was quenched with water and then extracted with ethyl acetate. The organic layer was dried over anhydrous $MgSO_4$ and evaporated under reduced pressure. The crude compound was purified by column chromatography on silica gel ($CH_2Cl_2/Et_2O$, 6/4) to afford the corresponding compound **3** as an orange solid (90.3 mg, 90%).

$^1$H (300 MHz, $CDCl_3$) δ 8.94 ppm (s, 1H), 7.70-7.64 (m, 2H), 7.45-7.37 (m, 3H), 7.20-7.17 (m, 2H), 6.75 (d, *J* = 8.1 Hz, 1H), 6.63 (d, *J* = 9.6 Hz, 1H), 4.53 (s, 2H), 3.45 (s, 3H). All the physical and spectroscopic data were in complete agreement with the reported ones **[42].**

8-((dimethylamino)methyl)-6-methoxy-7-phenyl-1*H*-phenalen-1-one **4**

Under argon atmosphere, 8-(hydroxymethyl)-6-methoxy-7-phenyl-1*H*-phenalen-1-one **3** (100 mg, 0.32 mmol, 1 equiv.) was introduced in anhydrous dichloromethane (2.4 mL). Triethylamine (89 μmol, 0.64 mmol, 2 equiv.) and methanesulfonyl chloride (29 μmol, 0.38 mmol, 1.2 equiv.) were added at 0°C. The mixture was stirred 30 minutes and dimethylamine (2 M in THF, 0.7 mL, 1.4 mmol, 4.4 equiv.) was then added. The reaction mixture was allowed to warm to room temperature and was left to stir for 1 hour, at which point water was added. The mixture was then extracted with ethyl acetate. The organic layer was dried over anhydrous $Na_2SO_4$ and evaporated under reduced pressure to afford the corresponding product **4** as a yellow solid (72.3 mg, 67%).

mp = 201-204°C. $^1$H NMR (400 MHz, $CDCl_3$) δ 8.94 ppm (s, 1H), 7.70 (d, *J* = 9.7 Hz, 1H), 7.67 (d, *J* = 8.0 Hz, 1H), 7.44-7.36 (m, 3H), 7.20-7.18 (m, 2H), 6.77 (d, *J* = 8.0 Hz, 1H), 6.65 (d, *J* = 9.6 Hz, 1H), 3.44 (s, 5H), 2.24 (s, 6H). $^{13}$C NMR (100 MHz, $CDCl_3$) δ 185.6 ppm, 161.6, 145.8, 142.4, 141.7, 133.6, 132.8, 129.2, 128.8, 128.3 (2C), 127.5 (3C), 126.7, 126.1, 123.9, 121.1, 106.5, 60.3, 55.7, 45.1 (2C). IR (ATR, $cm^{-1}$) 2821, 1635, 1562, 1508, 848, 703. HRMS (ES+) m/z calculated for $C_{23}H_{22}NO_2$ $[M+H]^+$ 344.1651; found 344.1638.

8-(chloromethyl)-6-methoxy-7-phenyl-1*H*-phenalen-1-one **5**

Under an argon atmosphere, 8-(hydroxymethyl)-6-methoxy-7-phenyl-1*H*-phenalen-1-one **3** (100.9 mg, 0.32 mmol, 1 equiv.), anhydrous triethylamine (88 μL, 0.64 mmol, 2 equiv.) and then *p*-toluenesulfonyl chloride (91 mg, 0.48 mmol, 1.5 equiv.) were introduced in anhydrous dichloromethane (1.5 mL). The reaction mixture was left to stir for 24 hours at room temperature, at which point a brine solution was added. The mixture was then extracted with dichloromethane. The organic layer was dried over anhydrous $MgSO_4$ and evaporated under reduced pressure. The crude compound was purified by column chromatography on silica gel (Cyclohexane/EtOAc, 6/4) to afford the corresponding product **5** as a yellow solid (94.4 mg, 88%).
$^1H$ (400 MHz, $CDCl_3$) δ 8.84 ppm (s, 1H), 7.72 (d, *J* = 9.7 Hz, 1H), 7.69 (d, *J* = 8.1 Hz, 1H), 7.49-7.35 (m, 3H), 7.25-7.21 (m, 2H), 6.78 (d, *J* = 8.1 Hz, 1H), 6.65 (d, *J* = 9.7 Hz, 1H), 4.47 (s, 2H), 3.46 (s, 3H). All the physical and spectroscopic data were in complete agreement with the reported ones **[42].**

*N*-benzyl-1-(6-methoxy-1-oxo-7-phenyl-1*H*-phenalen-8-yl)-*N*,*N*-dimethylmethanaminium chloride **1a**

Under argon atmosphere, 8-((dimethylamino)methyl)-6-methoxy-7-phenyl-1*H*-phenalen-1-one **4** (152.6 mg, 0.44 mmol, 1 equiv.), and (chloromethyl)benzene (581 μL, 4.44 mmol, 10 equiv.) were introduced in anhydrous dichloromethane (2.2 mL). The reaction mixture was refluxed for 12 hours. After cooling, the solvent was removed under reduced pressure. The solid was suspended in toluene and introduced into an Eppendorf tube, sonicated for 30 seconds, and centrifuged to remove the supernatant. This washing step was repeated 4 times. The solid was then dried under reduced pressure to afford the corresponding product **1a** as an orange solid (204.6 mg, 98%).
$^1H$ NMR (300 MHz, $D_2O$) δ 8.26 ppm (s, 1H), 7.65 (d, *J* = 8.2 Hz, 1H), 7.62 (d, *J* = 9.6 Hz, 1H), 7.58-7.55 (m, 1H), 7.45 (app. t, *J* = 7.6 Hz, 2H), 7.34-7.23 (m, 3H), 7.19 (app. t, *J* = 7.6 Hz, 2H), 6.79 (d, *J* = 8.2 Hz, 1H), 6.69-6.56 (m, 2H), 6.38 (d, *J* = 9.6 Hz, 1H), 4.36 (s, 2H), 4.35 (s, 2H), 3.32 (s, 3H), 2.58 (s, 6H). $^{13}C$ NMR (75 MHz, $D_2O$) δ 186.0 ppm, 162.5, 150.1, 145.4, 138.4, 138.3, 134.6, 132.7 (2C), 131.1, 129.4 (2C), 128.5 (2C), 128.4, 128.0, 127.9 (2C), 127.4, 126.5, 124.1, 123.7, 123.2, 119.5, 108.6, 70.3, 61.5, 56.0, 49.2 (2C). IR (ATR, $cm^{-1}$) 3348, 3023, 2360, 1635, 1571, 1458, 1104, 839, 702. HRMS (ES+) m/z calculated for $C_{30}H_{28}NO_2$ [M+] 434.2120; found 434.2110.

1-((6-methoxy-1-oxo-7-phenyl-1*H*-phenalen-8-yl)methyl)pyridin-1-ium chloride **1b**

Under argon atmosphere, 8-(chloromethyl)-6-methoxy-7-phenyl-1*H*-phenalen-1-one **5** (150 mg, 0.448 mmol, 1 equiv.) and anhydrous pyridine (361 μL, 4.48 mmol, 10 equiv.) were added in anhydrous dichloromethane (2.5 mL). The reaction mixture was refluxed for 16 hours. After cooling, the solvent was removed under reduced pressure. The solid was suspended in diethyl ether and introduced into an Eppendorf tube, sonicated for 30 seconds, and centrifuged to remove the supernatant. This washing step was repeated 4 times. The solid was then dried under reduced pressure to afford the corresponding product **1b** as an orange solid (161.9 mg, 88%).
$^1H$ NMR (400 MHz, $D_2O$) δ 8.48 ppm (tt, *J* = 7.8, 1.3 Hz, 1H), 8.47 (s, 1H), 8.12-8.10 (m, 2H), 7.88-7.84 (m, 2H), 7.54 (tt, *J* = 7.8, 1.3 Hz, 1H), 7.54 (d, *J* = 8.3 Hz, 1H), 7.49 (d, *J* = 9.7 Hz, 1H), 7.43 (app. t, *J* = 7.3 Hz, 2H), 6.67-6.73 (m, 3H), 6.30 (d, *J* = 9.7 Hz, 1H), 5.78 (s, 2H), 3.40 (s, 3H). $^{13}C$ NMR (100 MHz, $D_2O$) δ 185.8 ppm, 162.4, 148.2, 145.8, 144.8, 143.9 (2C), 139.2, 138.1, 133.1, 129.6, 128.8 (2C), 128.3, 128.0 (3C), 127.8, 126.6 (2C), 123.6, 123.1, 119.3, 108.2, 63.0, 56.0. IR (ATR, $cm^{-1}$) 3393, 3057, 1634, 1575, 1486, 835, 701. HRMS (ES+) m/z calculated for $C_{26}H_{20}NO_2$ [M+] 378.1493; found 378.1494.

8-((bis(2-hydroxyethyl)amino)methyl)-6-methoxy-7-phenyl-1*H*-phenalen-1-one chloride **1c**

Under argon atmosphere, 8-(chloromethyl)-6-methoxy-7-phenyl-1*H*-phenalen-1-one **5** (70 mg, 0.21 mmol, 1 equiv.), and *N*-methyldiethanolamine (121 μL, 1.05 mmol, 5 equiv.) were introduced in anhydrous tetrahydrofuran (1.1 mL). The reaction mixture was refluxed for 16 hours. After cooling, the solvent was removed under reduced pressure. The solid was suspended in tetrahydrofuran and introduced into an Eppendorf tube, sonicated for 30 seconds, and centrifuged to remove the supernatant. This washing step was repeated 3 times. This purification step was repeated with toluene. The solid was then dried under reduced pressure to afford the corresponding product **1c** as an orange solid (83.8 mg, 89%). $^1H$ NMR (400 MHz, $D_2O$) δ 8.4-8.2 ppm (m, 1H), 7.7-7.4 (m, 5H), 7.02-6.93 (m, 2H), 6.9-6.7 (m, 1H), 6.3-6.2 (m, 1H), 4.77 (s, 2H), 3.77 (app. t, *J* = 4.8 Hz, 4H), 3.39 (s, 3H), 3.29 (qd, *J* = 13.8, 6.6 Hz, 4H), 2.67 (s, 3H). $^{13}C$ NMR (100 MHz, $D_2O$) δ 185.8 ppm, 162.4, 150.1, 145.1, 138.8, 138.2, 134.4, 129.0 (2C), 128.2 (3C), 128.1, 127.2, 124.2, 123.5, 123.1, 119.2, 108.4, 63.5 (3C), 55.9, 55.1 (2C), 48.3. HRMS (ES+) m/z calculated for $C_{26}H_{28}NO_4$ [M+] 418.2018; found 418.2011.

## Example 2: Results and discussion

- ***Photophysical characterizations***

***UV/Visible and fluorescence spectroscopy:*** UV-Vis spectra were recorded on an Agilent 8453 Diode-Array spectrophotometer in the range of 250-700 nm. Emission spectra were measured in an ISS PC1 spectrofluorometer at room temperature. Fluorescence quantum yields were determined using equation (1) and 6-ethoxy-phenalenone in methanol as reference **[35].**

$$\Phi_{F,sample} = \Phi_{F,reference} \frac{A_{reference}}{A_{sample}} \frac{I_{sample}}{I_{reference}} \left(\frac{\eta_{sample}}{\eta_{reference}}\right)^2 (1)$$

$\Phi_F$ is the fluorescence quantum yields. A: is the absorbance in the *wavelength* of excitation, I correspond to area of the emission spectra and $\eta$ is the refractive index of the solvent. The excitation wavelength was 400 nm for all steady state fluorescence experiments.

*Luminescence* lifetime measurements were carried out using a PicoQuant FluoTime 200 fluorescence lifetime spectrometer with a multichannel scaler (PicoQuant's Timeharp 250) with the time correlated single photon counting (TCSPC) method. As excitation resource laser 405 nm was employed.

***Singlet oxygen quantum yield measurements:*** Lifetime decays of singlet oxygen, $O_2(^1\Delta_g)$, were acquired with a FluoTime 200 equipped with multichannel scaler Nanoharp 200. Excitation at 355 nm was achieved with a laser FTSS355-Q3, (Crystal Laser, Berlin, Germany) working at 1 kHz repetition rate. Excitation at 473nm was achieved with a CNI laser AO-V-473 (Changchun New Industries Optoelectronics Technology Co. Ltd., China), working at 1.5 kHz repetition rate. For the detection at 1270 nm a NIR PMT H10330A (Hamamatsu) was employed. The $O_2(^1\Delta_g)$ quantum yields ($\Phi_\Delta$) were determined by comparing the intensity at zero time of the 1270 nm signals to those of optically-matched solutions of SAPYR or Eosin Y as references.

***Chemical trapping experiments:*** Quantum yields of singlet oxygen produced by **1a-c** was evaluated by monitoring the UV/Visible absorption change in an Agilent 8453 Diode-Array spectrophotometer of 3,3'-anthracene-9,10-diyldipropanoic acid (ADPA) which is a well-known singlet oxygen trap. Excitation was achieved at 473nm with a CNI laser AO-V-473 (Changchun New Industries Optoelectronics Technology Co. Ltd., China), working at 1.5 kHz repetition rate. A solution of **1a-c** in deuterated water ($A_{473nm}$ = 0.06) was prepared and an aliquot of ADPA in deuterated water was added to reach $A_{378nm}$ = 0.74. The photoabsorption spectral change of ADPA solution with photosensitizers **1a-c** was monitored following the decrease of the band at 382 nm. Plots of $Ln(A/A_0)$ vs. time (s) give linear graphs for which the slope is the experimental kinetic rate constant ($k$, $s^{-1}$) (Ao is the initial absorption intensity and A is the absorption intensity at the time *t*). From comparison of experimental rate constants between phenalenone derivatives **1a-c** and two selected actinometers (Eosin Y and Riboflavin, see supporting information for results using Riboflavin) using optically-matched solutions, singlet oxygen quantum yields of the photosensitizers $\Phi_\Delta$ can be determined from the following equation:

$$\Phi_\Delta = \frac{k}{k_{ref}} \times \Phi_{\Delta,\text{ref}}$$

***Photochemical stability:*** In an NMR tube, a solution of dyes **1a-c** or SAPYR in $D_2O$ ([C] = 17.5 mmol/L) was irradiated at 470 nm (3.5 mW.cm$^{-2}$) under oxygen or Argon atmosphere (the gas was bublled solution was oxygenated or degassed by bubbling through the solution for 5 minutes and put under gas atmosphere). 1H NMR spectra were recorded after 24h irradiation time for each sample.

- ***Antibacterial photodynamic inactivation studies***

To evaluate the biological activity of the molecules, the minimal inhibitory concentration (MIC) and the minimal bactericidal concentration (MBC) were *determined* on bacterial cultures (*Staphylococcus aureus* CIP76.25, *Pseudomonas aeruginosa* CIP76.110) by micromethod in 96-well plates. Briefly, bacterial cultures in log-phase were diluted in sterile PBS 1X at a concentration equivalent to 4×10^6 UFC/mL. Serial dilutions of different molecules were performed in PBS 1X to a final concentration from 200 to 1.5 μM and 50 μl of each dilution were added in the microplate wells. Then, 50 μl of diluted bacterial culture were added in each well. Two different plates for each strain were prepared: one plate for the dark condition and a second plate for light irradiation.

Two controls were included; a bacterial growth control with no molecule added and a negative control with PBS

without bacterial inoculum and no molecules.

The microplate was incubated at 37°C for 1h in dark condition. After that, bacteria were irradiated with LED white light (total fluence: 25J/$cm^2$). During the time of irradiation, the second plate was maintained in the dark. Then, 100 μL of 2-fold concentrated TS broth were added in each well and the microplate was incubated at 37°C for 24h. The MIC values were determined as the lowest concentration without observed bacterial growth. For determination of the MBC, 100 μl from wells where no evident growth was observed were diluted in physiological water (NaCl 0.9%). All 10-fold serial dilutions were spread on TS agar plates using an automatic plater (easySPIRAL®, Interscience, Saint-Nom La Bretêche, France). After incubation at 37 °C for 24 h, colonies were counted to determine total CFU per mL (CFU/mL). The MBC corresponds to the concentration of the active compound for which 99.99% of the bacteria have been killed (i.e. 4 log reduction compared to the untreated control). Three independent experiments were performed with each strain.

- ***Results***

**Photophysical Characterization**

Absorption and fluorescence spectra of photosensitizers **1a-c** were performed in different polar solvents (Figures 1, 2). The spectroscopic characterization of **1a-c** displayed a broad band in the 400-520 nm region and a weak absorption band around 355 nm. The signatures of **1a-c** are in line with previous studies about photophysical characterizations of 6-alkoxyphenalenones such as **2 [31,35].** Compared to phenalenone **2,** the introduction of positive charged moieties not directly connected to the phenalenone framework has a low impact on the absorption profile ($\Delta\lambda_{max}$ in MeOH < 10 nm). Switching from DMSO to methanol has a negligible influence on the longest-wavelength absorption maximum of **1a-c** while the use of water induced a red shift of absorption wavelength (+18-19 nm, Table 1).

Fluorescence spectra recorded at room temperature were shown in Figure 2 and the data were collected in Table 1. Dyes **1a-c** are characterized by fluorescence emission in the yellow spectral range ($\lambda_{em}$ = 533-551 nm) with large Stokes shifts ($\Delta\lambda$ = 88-96 nm) regardless of the solvents. The development of fluorescent organic dyes with large Stokes shifts ($\Delta\lambda$ > 80 nm) still attracts considerable interest for biological imaging owing to the reduction of the overlap between the absorption and emission spectra which lowers the interferences **[36].** A slight red shift of the emission was observed from DMSO to methanol and water and the emission intensities are higher in methanol and water with promising emission properties in the yellow spectral range. As expected, the fluorescence lifetimes determined at room temperature were short (5-8 ns) and consistent with previous studies dealing with photophysical characterizations of non-hydrophilic phenalenones **[31].**

**Table 1.** Photophysical *properties of the photosensitizers in different solvents*

| | Solvent | $\lambda_{abs}$ (nm) | $\varepsilon$ ($M^{-1}$ $cm^{-1}$)[a] | $\lambda_{max}$(em, nm)[b] | $\Delta\lambda$ (nm)[c] | $\Phi_F$[d] | TF (ns)[e] |
|---|---|---|---|---|---|---|---|
| **1a** | DMSO | 441 | 11426 | 533 | 92 | 0.069 | 6.04 |
| | MeOH | 447 | 9363 | 542 | 95 | 0.221 | 8.15 |
| | $H_2O$ | 459 | 9239 | 549 | 90 | 0.241 | 7.65 |
| **1b** | DMSO | 444 | 10472 | 535 | 91 | 0.067 | 6.24 |
| | MeOH | 447 | 9344 | 542 | 95 | 0.209 | 7.65 |
| | $H_2O$ | 463 | 9259 | 551 | 88 | 0.192 | 7.15 |
| **1c** | DMSO | 441 | 11984 | 533 | 92 | 0.071 | 5.38 |
| | MeOH | 446 | 12256 | 542 | 96 | 0.233 | 8.24 |
| | $H_2O$ | 459 | 10053 | 551 | 92 | 0.217 | 7.39 |

[a] Molar extinction coefficients were measured at the longest-wavelength absorption maximum. [b] Excited at 400 nm. [c] Stokes shift. [d] Fluorescence quantum yield using 6-ethoxyphenalenone as an actinometer. [e] Singlet lifetime.

High photostability of photosensitizers is an important criterion for practical aPDT applications. The study of the photochemical stability has been monitored by $^1H$ NMR spectroscopy upon blue light irradiation (470 nm, 3.5 mW.$cm^{-2}$) for 24h of solutions of **1a-c** in oxygenated deuterated water and deuterated water under Argon atmosphere. The results were compared to SAPYR which is a potent and well-established photosensitizer eliciting phototoxic activities against bacteria **[27-28].** Upon irradiation at 470 nm, dyes **1a-c** turned out to be stable with no change in the $^1H$ NMR spectra

while complete decomposition of SAPYR was observed under Argon and oxygen suggesting a higher photostability of dyes **1a-c** than SAPYR:

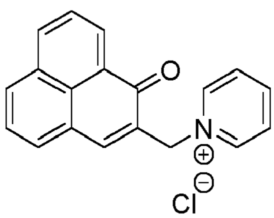

*SAPYR dye* **[27-28, 44]**

While dyes **1a-c** display good emission yields in methanol and water, phenalenone derivatives have generally low fluorescent quantum yields due to a very efficient intersystem crossing producing the corresponding triplet state involved in the production of singlet oxygen **[37].** Therefore, the ability of dyes **1a-c** to produce singlet oxygen was then investigated upon light illumination. Two techniques are commonly used for determining singlet oxygen quantum yields. The first strategy for quantifying singlet oxygen involves the use of a specific chemical probe whose reaction with singlet oxygen can be monitored by spectroscopy or other analytical techniques. The second approach is based on the direct observation of $^1O_2$ phosphorescence emission band at around 1270 nm. Both techniques have been used for determining singlet oxygen quantum yields of **1a-c** in selected solvents and the data are collected in Table 2.

**Table 2.** *Singlet oxygen quantum yields of photosensitizers* ***1a-c***

| | $\Phi_\Delta{}^a$ | | | | |
|---|---|---|---|---|---|
| | DMSO | MeOH | $D_2O$ | $D_2O$ (470 nm)[b] | $D_2O$ (470 nm)[c] |
| **1a** | 0.79 | 0.10 | 0.06 | 0.12 | 0.09 |
| **1b** | 0.63 | 0.12 | 0.06 | 0.11 | 0.15 |
| **1c** | 0.91 | 0.15 | 0.06 | 0.09 | 0.08 |

[a] Unless otherwise noted, singlet oxygen quantum yields have been determined by $^1O_2$ phosphorescence method using SAPYR as an actinometer ($\Phi_\Delta$ = 1.02) **[27]** with excitation at 355 nm. [b] Eosin Y was used as an actinometer ($\Phi_\Delta$ = 0.58) **[43]** with excitation at 470 nm. [c] Relative singlet oxygen quantum yields using 3,3'-anthracene-9,10-diyldipropanoic acid (ADPA) as the chemical probe and Eosin Y as the reference with excitation at 470 nm.

Singlet oxygen quantum yields measured in DMSO revealed the high propensity of photosensitizers **1a-c** to produce singlet oxygen. The best singlet oxygen quantum yield was found for **1c** ($\Phi_\Delta$ = 0.91 in DMSO) with respect to SAPYR ($\Phi_\Delta$ = 1.02) **[27]** under excitation at 355 nm. Switching from DMSO to methanol has a dramatic impact on singlet oxygen production with quantum yields between 0.10 and 0.15 for **1a-c.** The increase of the fluorescence observed in protic solvent (MeOH, $H_2O$ - Table 1) could explain the low production of singlet oxygen. Due to the short lifetime of singlet oxygen in water which makes difficult the detection of the phosphorescence signal at 1270 nm ($\tau_\Delta$ = 3.45 $\mu$s at 25°C) **[38],** the phosphorescence experiments have been carried out in $D_2O$ ($\tau_\Delta$ = 67.9 $\mu$s at 25°C) for which the non-radiative decay rate towards ground state oxygen is slower. The singlet oxygen quantum yields in $D_2O$ are comparable with those obtained in methanol. Dyes **1a-c** does not deviate from the Kasha-Vavilov rule because excitation wavelengths has no influence on singlet oxygen quantum yields with similar values obtained under excitation at 355 nm and 470 nm in $D_2O$ **[39].** The singlet oxygen quantum yields obtained by phosphorescence were compared with the chemical probe method using 3,3'-anthracene-9,10-diyldipropanoic acid (ADPA) as the trapping agent **[40].** Upon excitation at 470 nm, the change of absorbance of ADPA due to singlet oxygen trapping was monitored by UV/visible spectroscopy and the results gave similar singlet oxygen quantum yield ($\Phi_\Delta$ = 0.08-0.15 using Eosin Y as reference) than those obtained by phosphorescence method.

**Antibacterial photodynamic inactivation studies**

The photodynamic activities of **1a-c** was investigated against Gram-positive (*Staphylococcus aureus*) and Gram-negative bacteria (*Pseudomonas aeruginosa*) belonging to the ESKAPE bacteria group and for which new therapeutic solutions must be found due to their ability to counter the antibacterial action of common medicines **[41].** The chemical structure of the photosensitizers plays a crucial role in their bactericidal ability and therefore, the results were

compared to the pyridinium-based phenalenone SAPYR known to exhibit a strong phototoxicity against diverse bacterial strains (Table 3).

**Table 3.** *Minimum Inhibitory Concentration (MIC, values in μM) and Minimum Bactericidal Concentration (MBC, values in μM) of SAPYR and **1a-c** tested against 2 bacterial strains (S.a.: Staphylococcus aureus CIP76.25, P.a.: Pseudomonas aeruginosa CIP76.110) under dark (D) and light (L) conditions.*

| PS | *P.a* | | | | *S.a* | | | |
|---|---|---|---|---|---|---|---|---|
| | *D* | | *L* | | *D* | | *L* | |
| | MIC | MBC | MIC | MBC | MIC | MBC | MIC | MBC |
| SAPYR | > 200 | > 200 | > 200 | > 200 | > 200 | > 200 | 83.3 | 167 |
| **1a** | > 200 | > 200 | > 200 | > 200 | 167 | > 200 | 50 | 133 |
| **1b** | > 200 | > 200 | > 200 | > 200 | > 200 | > 200 | 50 | 67 |
| **1c** | > 200 | > 200 | 33 | 41.7 | > 200 | > 200 | 41.7 | 41.7 |

Minimum *Inhibitory* Concentration (MIC) defined as the lowest concentration of photosensitizer that prevents visible bacterial growth and Minimum Bactericidal Concentration (MBC) which is the lowest concentration that allowed no microbial growth are collected in Table 3. The antibacterial tests have been carried out under dark conditions and upon illumination with white light LED (total fluence: 25 J/cm$^2$) for 6 hours. Under dark conditions, no significant activity was observed for the photosensitizers against the strains, except for **1a** with a MIC value of 167 μM. Under light irradiation, bactericidal activities at the micromolar range were observed with different values depending on the structures of the photosensitizers. Therefore, light is essential to the photoinactivation of the bacteria which demonstrates the absence of dark toxicity. Interestingly, the MIC and MBC values showed photosensitizers **1a-c** were more effective against *Staphylococcus aureus* than the reference compound SAPYR. In addition, higher activities were observed against Gram-positive bacteria (*Staphylococcus aureus*) than Gram-negative bacteria (*Pseudomonas aeruginosa*) owing to a different membrane structure which makes Gram-negative bacteria less prone to aPDT treatment **[22,34,42].** Comparison of the antibacterial activity of SAPYR and **1b** possessing both a pyridinium moiety showed that not only quaternarization but also the structure of the phenalenone framework and the position of the cationic group plays an important role in their ability to eradicate bacteria. Compared to SAPYR, photosensitizer **1b** was more effective against *Staphylococcus aureus* and the best results were obtained with photosensitizer **1c.** Upon illumination under white light, SAPYR and dyes **1a-b** did not exhibit any photodynamic effects against *Pseudomonas aeruginosa* while **1c** was effective against *Staphylococcus aureus* (MIC 41.7 μM) and *Pseudomonas aeruginosa* (MIC 33 μM)..

**Conclusion**

In summary, herein is reported the synthesis of new hydrophilic phenalenone-based photosensitizers containing a quaternary ammonium or a pyridinium unit. All derivatives produce singlet oxygen and exhibit yellow fluorescence upon blue light irradiation. Antibacterial photodynamic inactivation studies revealed promising phototoxicity activities of the photosensitizers against Gram-positive *(Staphylococcus aureus)* and Gram-negative bacteria (*Pseudomonas aeruginosa*).

**List of references**

**(1)** Cars, O.; Chandy, S. J.; Mpundu, M.; Peralta, A. Q.; Zorzet, A.; So, A. D. Resetting the Agenda for Antibiotic Resistance through a Health Systems Perspective. Lancet Glob. Health 2021, 9, e1022-e1027.

**(2)** Levy, S. B.; Marshall, B. Antibacterial Resistance Worldwide: Causes, Challenges and Responses. Nat. Med. 2004, 10, S122-S129.

**(3)** UN Interagency Coordination Group on Antimicrobial Resistance. No time to wait: securing the future from drug-resistant infections, World Health Organization, Geneva 2019**.**

**(4)** McManus, M. C. Mechanisms of Bacterial Resistance to Antimicrobial Agents. Am. J. Health. Syst. Pharm. 1997, 54, 1420-1433.

**(5)** Llor, C.; Bjerrum, L. Antimicrobial Resistance: Risk Associated with Antibiotic Overuse and Initiatives to Reduce the Problem. Ther. Adv. Drug Saf. 2014, 5, 229-241.

**(6)** Jia, Q.; Song, Q.; Li, P.; Huang, W. Rejuvenated Photodynamic Therapy for Bacterial Infections. Adv. Healthc. Mater. 2019, 8, 1900608.

**(7)** Cieplik, F.; Deng, D.; Crielaard, W.; Buchalla, W.; Hellwig, E.; Al-Ahmad, A.; Maisch, T. Antimicrobial Photodynamic Therapy - What We Know and What We Don't. Crit. Rev. Microbiol. 2018, 44, 571-589.

**(8)** Hamblin, M. R.; Hasan, T. Photodynamic Therapy: A New Antimicrobial Approach to Infectious Disease? Photochem. Photobiol. Sci. 2004, 3, 436-450.

**(9)** Singlet Oxygen: Applications in Biosciences and Nanosciences; Nonell, S., Flors, C., Eds.; Comprehensive Series in Photochemical & Photobiological Sciences; The Royal Society of Chemistry, 2016.

**(10)** Quina, F. H.; Silva, G. T. M. The Photophysics of Photosensitization: A Brief Overview. J. Photochem. Photobiol. 2021, 7, 100042.

**(11)** Sasikumar, D.; John, A. T.; Sunny, J.; Hariharan, M. Access to the Triplet Excited States of Organic Chromophores. Chem Soc Rev 2020, 49, 6122-6140.

**(12)** Baptista, M. S.; Cadet, J.; Di Mascio, P.; Ghogare, A. A.; Greer, A.; Hamblin, M. R.; Lorente, C.; Nunez, S. C.; Ribeiro, M. S.; Thomas, A. H.; Vignoni, M.; Yoshimura, T. M. Type I and Type II Photosensitized Oxidation Reactions: Guidelines and Mechanistic Pathways. Photochem. Photobiol. 2017, 93, 912-919.

**(13)** Almeida, A.; Faustino, M. A.; Tomé, J. P. Photodynamic Inactivation of Bacteria: Finding the Effective Targets. Future Med. Chem. 2015, 7, 1221-1224.

**(14)** Alves, E.; Faustino, M. A.; Neves, M. G.; Cunha, A.; Tome, J.; Almeida, A. An Insight on Bacterial Cellular Targets of Photodynamic Inactivation. Future Med. Chem. 2014, 6, 141-164.

**(15)** Marasini, S.; Leanse, L. G.; Dai, T. Can Microorganisms Develop Resistance against Light Based Anti-Infective Agents? Adv. Drug Deliv. Rev. 2021, 175, 113822.

**(16)** Pham, T. C.; Nguyen, V.-N.; Choi, Y.; Lee, S.; Yoon, J. Recent Strategies to Develop Innovative Photosensitizers for Enhanced Photodynamic Therapy. Chem. Rev. 2021, 121, 13454-13619.

**(17)** Maisch, T. Photoantimicrobials-An Update. Transl. Biophotonics 2020, 2, e201900033.

**(18)** Schmidt, R.; Tanielian, C.; Dunsbach, R.; Wolff, C. Phenalenone, a Universal Reference Compound for the Determination of Quantum Yields of Singlet Oxygen O2(1Δg) Sensitization. J. Photochem. Photobiol. Chem. 1994, 79, 11-17.

**(19)** Martí, C.; Jürgens, O.; Cuenca, O.; Casals, M.; Nonell, S. Aromatic Ketones as Standards for Singlet Molecular Oxygen O2(1Δg) Photosensitization. Time-Resolved Photoacoustic and near-IR Emission Studies. J. Photochem. Photobiol. Chem. 1996, 97, 11-18.

**(20)** Oliveros, E.; H. Bossmann, S.; Nonell, S.; Martí, C.; Heit, G.; Tröscher, G.; Neuner, A.; Martinez, C.; M. Braun, A. Photochemistry of the Singlet Oxygen [O2(1Δg)] Sensitizer Perinaphthenone (Phenalenone) in N,N'-Dimethylacetamide and 1,4-Dioxane. New J Chem 1999, 23, 85-93.

**(21)** Flors, C.; Nonell, S. Light and Singlet Oxygen in Plant Defense Against Pathogens: Phototoxic Phenalenone Phytoalexins. Acc. Chem. Res. 2006, 39, 293-300.

**(22)** Godard, J.; Gibbons, D.; Leroy-Lhez, S.; Williams, R. M.; Villandier, N.; Ouk, T.-S.; Brégier, F.; Sol, V. Development of Phenalenone-Triazolium Salt Derivatives for APDT: Synthesis and Antibacterial Screening. Antibiotics 2021, 10, 626.

**(23)** Muehler, D.; Rupp, C. M.; Keceli, S.; Brochhausen, C.; Siegmund, H.; Maisch, T.; Hiller, K.-A.; Buchalla, W.; Cieplik, F. Insights Into Mechanisms of Antimicrobial Photodynamic Action Toward Biofilms Using Phenalen-1-One Derivatives as Photosensitizers. Front. Microbiol. 2020, 11, 2594.

**(24)** Cieplik, F.; Wimmer, F.; Muehler, D.; Thurnheer, T.; Belibasakis, G. N.; Hiller, K.-A.; Maisch, T.; Buchalla, W. Phenalen-1-One-Mediated Antimicrobial Photodynamic Therapy and Chlorhexidine Applied to a Novel Caries Biofilm Model. Caries Res. 2018, 52, 447-453.

**(25)** Muehler, D.; Sommer, K.; Wennige, S.; Hiller, K.-A.; Cieplik, F.; Maisch, T.; Späth, A. Light-Activated Phenalen-1-One Bactericides: Efficacy, Toxicity and Mechanism Compared with Benzalkonium Chloride. Future Microbiol. 2017, 12, 1297-1310.

**(26)** Tabenski, I.; Cieplik, F.; Tabenski, L.; Regensburger, J.; Hiller, K.-A.; Buchalla, W.; Maisch, T.; Späth, A. The Impact of Cationic Substituents in Phenalen-1-One Photosensitizers on Antimicrobial Photodynamic Efficacy. Photochem. Photobiol. Sci. 2016, 15, 57-68.

**(27)** Späth, A.; Leibl, C.; Cieplik, F.; Lehner, K.; Regensburger, J.; Hiller, K.-A.; Bäumler, W.; Schmalz, G.; Maisch, T. Improving Photodynamic Inactivation of Bacteria in Dentistry: Highly Effective and Fast Killing of Oral Key Pathogens with Novel Tooth-Colored Type-II Photosensitizers. J. Med. Chem. 2014, 57, 5157-5168.

**(28)** Cieplik, F.; Späth, A.; Regensburger, J.; Gollmer, A.; Tabenski, L.; Hiller, K.-A.; Bäumler, W.; Maisch, T.; Schmalz, G. Photodynamic Biofilm Inactivation by SAPYR-An Exclusive Singlet Oxygen Photosensitizer. Free Radic. Biol. Med. 2013, 65, 477-487.

**(29)** Bresolí-Obach, R.; Gispert, I.; Peña, D. G.; Boga, S.; Gulias, Ó.; Agut, M.; Vázquez, M. E.; Nonell, S. Triphenylphosphonium Cation: A Valuable Functional Group for Antimicrobial Photodynamic Therapy. J. Biophotonics

2018, 11, e201800054.
**(30)** Carden, R. G.; Sommers, K. J.; Schrank, C. L.; Leitgeb, A. J.; Feliciano, J. A.; Wuest, W. M.; Minbiole, K. P. C. Advancements in the Development of Non-Nitrogen-Based Amphiphilic Antiseptics to Overcome Pathogenic Bacterial Resistance. ChemMedChem 2020, 15, 1974-1984.
**(31)** De Bonfils, P.; Verron, E.; Sandoval-Altamirano, C.; Jaque, P.; Moreau, X.; Gunther, G.; Nun, P.; Coeffard, V. Unusual Oxidative Dealkylation Strategy toward Functionalized Phenalenones as Singlet Oxygen Photosensitizers and Photophysical Studies. J. Org. Chem. 2020, 85, 10603-10616 and references cited therein.
**(32)** For application of phenalenone photosensitizers in photodynamic cancer therapy, see: Kaye, E. G.; Kailass, K.; Sadovski, O.; Beharry, A. A. A Green-Absorbing, Red-Fluorescent Phenalenone-Based Photosensitizer as a Theranostic Agent for Photodynamic Therapy. ACS Med. Chem. Lett. 2021, 12, 1295-1301.
**(33)** Kharkwal, G. B.; Sharma, S. K.; Huang, Y.-Y.; Dai, T.; Hamblin, M. R. Photodynamic Therapy for Infections: Clinical Applications. Lasers Surg. Med. 2011, 43, 755-767.
**(34)** Lin, G.; Hu, M.; Zhang, R.; Zhu, Y.; Gu, K.; Bai, J.; Li, J.; Dong, X.; Zhao, W. Discovery of Meso-(Meta-Pyridinium) BODIPY Photosensitizers: In Vitro and In Vivo Evaluations for Antimicrobial Photodynamic Therapy. J. Med. Chem. 2021, 64, 18143-18157.
**(35)** Sandoval-Altamirano, C.; De la Fuente, J. R.; Berrios, E.; Sanchez, S. A.; Pizarro, N.; Morales, J.; Gunther, G. Photophysical Characterization of Hydroxy and Ethoxy Phenalenone Derivatives. J. Photochem. Photobiol. Chem. 2018, 353, 349-357.
**(36)** Lavis, L. D.; Raines, R. T. Bright Ideas for Chemical Biology. ACS Chem. Biol. 2008, 3 (3), 142-155.
**(37)** Daza, M. C.; Doerr, M.; Salzmann, S.; Marian, C. M.; Thiel, W. Photophysics of Phenalenone: Quantum-Mechanical Investigation of Singlet-Triplet Intersystem Crossing. Phys Chem Chem Phys 2009, 11, 1688-1696.
**(38)** Bregnhøj, M.; Westberg, M.; Jensen, F.; Ogilby, P. R. Solvent-Dependent Singlet Oxygen Lifetimes: Temperature Effects Implicate Tunneling and Charge-Transfer Interactions. Phys Chem Chem Phys 2016, 18, 22946-22961.
**(39)** Turro, N. J.; Ramamurthy, V.; Scaiano, J. C. Modern Molecular Photochemistry of Organic Molecules; University Science Books: Sausalito, CA, 2010.
**(40)** Kuznetsova, N. A.; Gretsova, N. S.; Yuzhakova, O. A.; Negrimovskii, V. M.; Kaliya, O. L.; Luk'yanets, E. A. New Reagents for Determination of the Quantum Efficiency of Singlet Oxygen Generation in Aqueous Media. Russ. J. Gen. Chem. 2001, 71, 36-41.
**(41)** Rice, L. B. Federal Funding for the Study of Antimicrobial Resistance in Nosocomial Pathogens: No ESKAPE. J. Infect. Dis. 2008, 197, 1079-1081.
**(42)** De Bonfils, P.; Verron, E.; Nun, P.; Coeffard, V. Photoinduced Storage and Thermal Release of Singlet Oxygen from 1,2-Dihydropyridine Endoperoxides. ChemPhotoChem 2021, 5, 847-856.
**(43)** WO2012/113860.
**(44)** WO2018/167264.

## Claims

1. Compound of formula I:

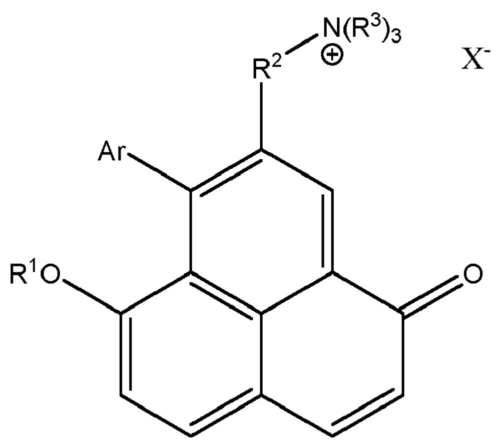

wherein,

- $R^1$ represents a $C_1$ to $C_{12}$ linear or branched, saturated or unsaturated, hydrocarbon chain, optionally bearing

a substituted or unsubstituted 4- to 26-membered aryl or heteroaryl moiety,
- Ar represents a substituted or unsubstituted 4- to 30-membered aryl or heteroaryl moiety,
- $R^2$ represents a $C_1$ to $C_3$ linear or branched alkyl chain,
- $X^-$ represents an anion,
- $R^3$ which independently of one another may be identical to or different from each other, respectively represent:

  - a $C_1$ to $C_{10}$ linear or branched alkyl chain, optionally bearing a substituted or unsubstituted, saturated or unsaturated, aromatic or non-aromatic, 4- to 26-membered cyclic or heterocyclic moiety,
  - a $C_1$ to $C_{26}$ linear or branched hydroxyalkyl group, and/or
  - at least two $R^3$ groups form, together with the nitrogen atom, a substituted or unsubstituted, saturated or unsaturated, aromatic or non-aromatic, 4- to 10-membered heterocyclic moiety.

**2.** Compound according to claim 1, wherein $R^1$ is chosen from a $C_1$ to $C_4$ saturated or unsaturated, hydrocarbon chain, a benzyl group and a methoxybenzyl group, preferably from methyl, ethyl, isopropyl, n-propyl, n-butyl, n-propenyl, n-propynyl, benzyl and methoxybenzyl .

**3.** Compound according to claim 1 or 2, wherein Ar is a substituted or unsubstituted 4- to 15-membered aryl or heteroaryl moiety, optionally substituted by one or two, identical or different, groups chosen from halo, - $N_3$, $-OR^4$, $-NO_2$, $-R^4$, -CN, -CHO, $-COR^4$, $-CO_2R^4$, phenyl, $2\text{-}R^4C_6H_4$ and 2-pyridyl, in which $R^4$ represents a $C_1$ to $C_3$ alkyl chain.

**4.** Compound according to any of preceding claims, wherein $R^2$ is $CH_2$.

**5.** Compound according to any of preceding claims, wherein the $R^3$ groups are independently chosen methyl, hydroxyethyl, C1 to C4 N-alkylpyridin-2(1H)-one, substituted or unsubstituted benzyl group, substituted or unsubstituted naphthalen-2-ylmethyl group and substituted or unsubstituted anthracen-2-ylmethyl group, or the $R^3$ groups form, together with the nitrogen atom, a substituted or unsubstituted heteroaryl group, preferably a pyridinyl group.

**6.** Pharmaceutical composition comprising a compound according to any of preceding claims, a solvent, preferably water and/or an organic solvent chosen from DMSO, MeOH, EtOH, MeCN and DMF, and optionally at least one pharmaceutically acceptable excipient.

**7.** Compound or composition according to any of preceding claims 1-6, for use as a medicine.

**8.** Compound or composition for use as preceding claim, wherein the medicine is an antimicrobial agent or a theranostic agent.

**9.** Compound or composition according to any of preceding claims 1-6, for use in antimicrobial photodynamic therapy.

**10.** Compound or composition according to the preceding claims 1-6, for use in the treatment of acne vulgaris, periodontis infections, rosacea, ptyriasis versicolor, molluscum contagiosum, genital herpes, tinea pedis, endodontics infections, viral keratitis, COVID-19 or influenza.

**11.** Compound or composition according to any of preceding claims 1 to 6, for use in odontology, preferably for use in the treatment of oral candidiasis, tooth decay and periodontitis.

**12.** Antimicrobial composition comprising a compound according to any of preceding claims 1 to 5, a solvent, preferably water and/or an organic solvent chosen from MeOH, EtOH, DMSO, DMF and MeCN, and optionally at least one excipient.

**13.** Use of a compound or composition according to any of preceding claims 1-5 and 12, as an antimicrobial agent.

**14.** Use according to the preceding claim for eradication of microbial biofilms from surfaces, preferably for the treatment of surfaces such as medical tools or any apparatus/tool/device found in a medical environment, for the treatment of paints for buildings or surfaces, or for application of antimicrobial photodynamic therapy in the food industry.

**15.** Use according to claim 13 for water depollution, preferably wastewater of drinkable water, more preferably for the destruction of fungi, bacteria, viruses, parasites or chemical compounds.

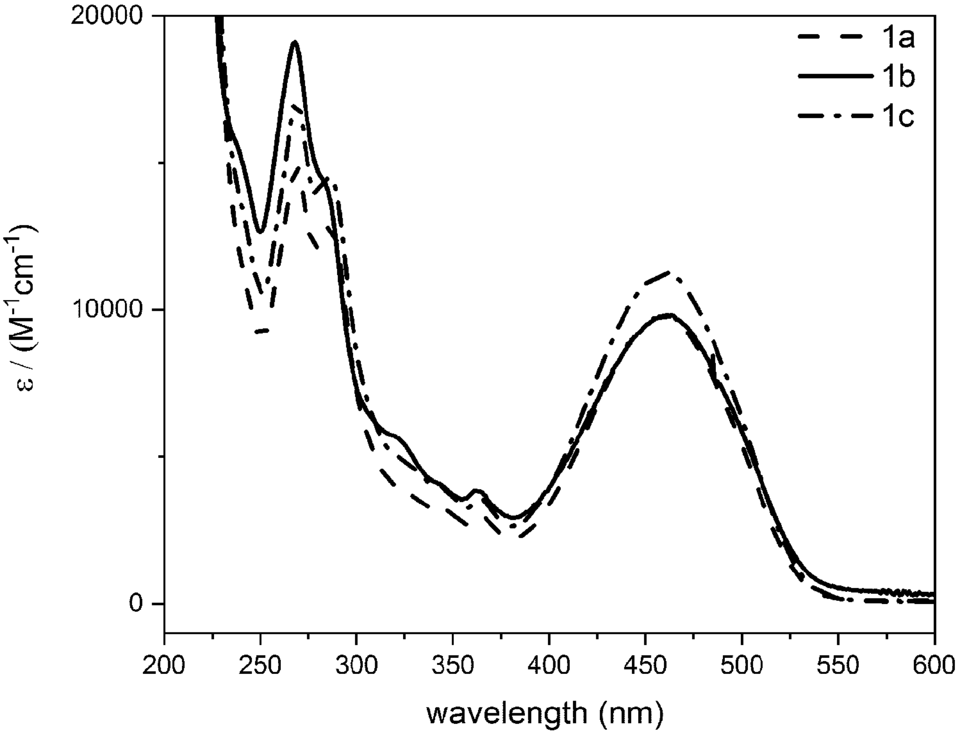


Figure 1

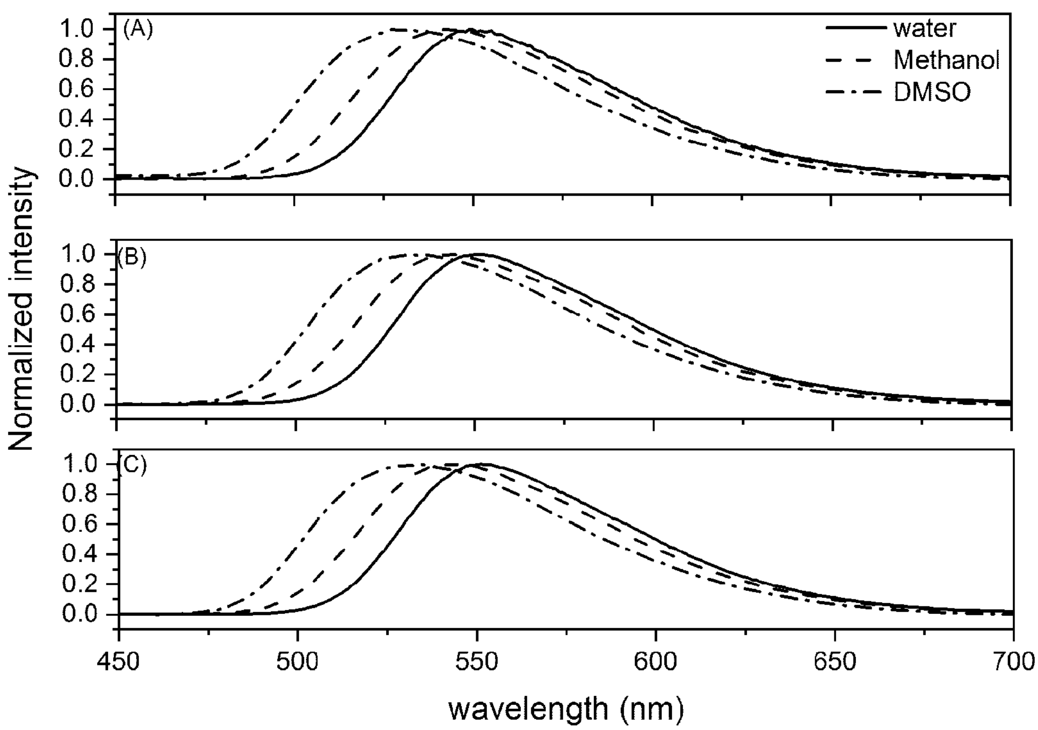


Figure 2

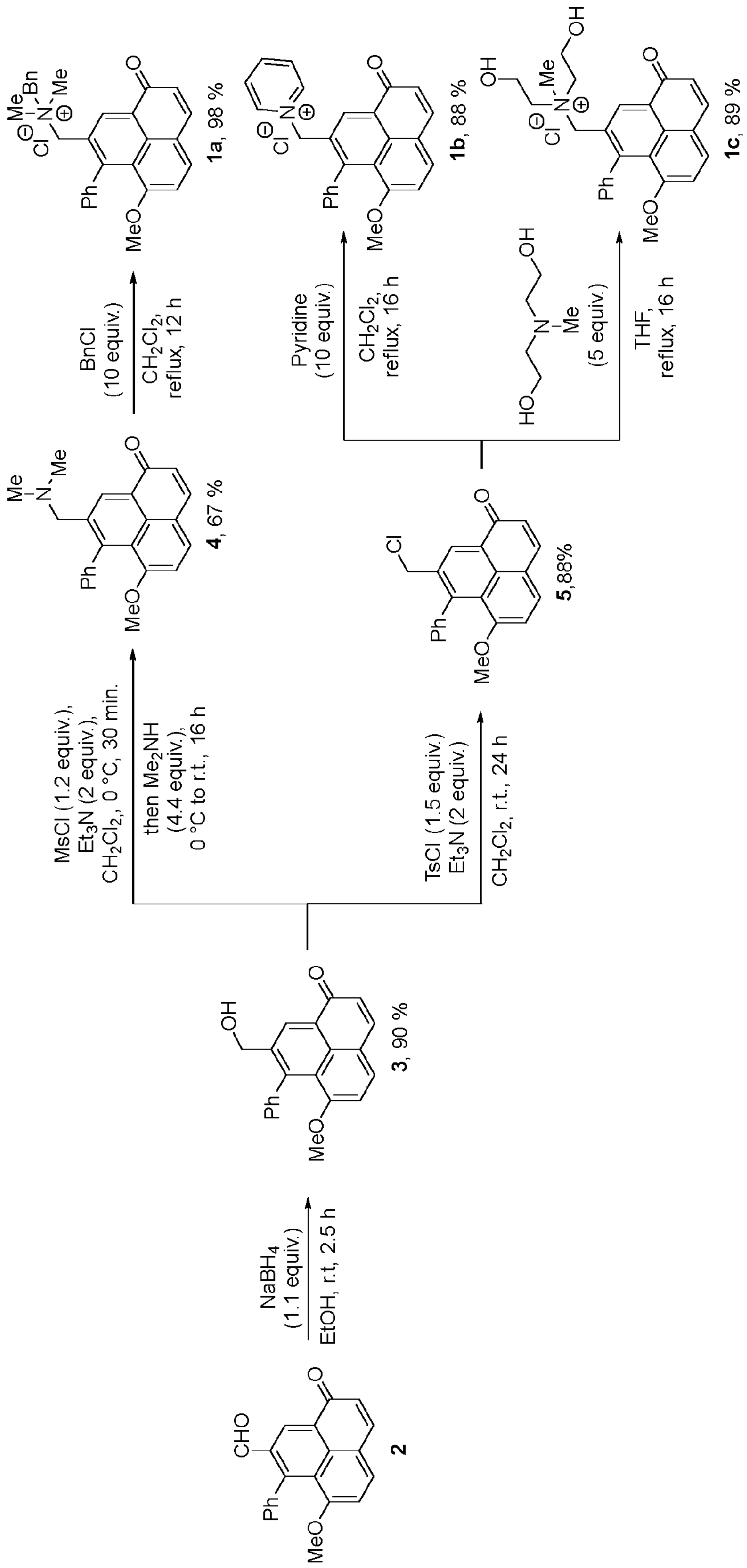

2
NaBH4
(1.1 equiv.)
EtOH, r.t, 2.5 h
3, 90 %
MsCl (1.2 equiv.),
Et3N (2 equiv.),
CH2Cl2, 0 °C, 30 min.
then Me2NH
(4.4 equiv.),
0 °C to r.t., 16 h
4, 67 %
BnCl
(10 equiv.)
CH2Cl2,
reflux, 12 h
1a, 98 %
TsCl (1.5 equiv.)
Et3N (2 equiv.)
CH2Cl2, r.t., 24 h
5,88%
Pyridine
(10 equiv.)
CH2Cl2,
reflux, 16 h
1b, 88 %
(5 equiv.)
THF,
reflux, 16 h
1c, 89 %


Figure 3

Europäisches Patentamt
European Patent Office
Office européen des brevets

# EUROPEAN SEARCH REPORT

Application Number
EP 22 30 5943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/039184 A1 (BAEUMLER WOLFGANG [DE] ET AL) 6 February 2014 (2014-02-06)<br>* page 30 - page 31; claims 1-2 *<br>----- | 1-15 | INV.<br>A61P31/00<br>A61L2/08<br>C09B57/00 |
| X | US 2020/087259 A1 (SPÄTH ANDREAS [DE] ET AL) 19 March 2020 (2020-03-19)<br>* page 78; claim 1 *<br>* page 102; claim 21 *<br>----- | 1-15 | |
| | | | TECHNICAL FIELDS SEARCHED (IPC) |
| | | | A61P<br>A61L<br>C09B |

The present search report has been drawn up for all claims

| Place of search | Date of completion of the search | Examiner |
|---|---|---|
| Munich | 19 December 2022 | Jeanjean, Fabien |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 30 5943

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2022

| Patent document cited in search report | Publication date | Patent family member(s) | Publication date |
|---|---|---|---|
| US 2014039184 A1 | 06-02-2014 | DE 102011012343 A1 | 30-08-2012 |
| | | EP 2678035 A2 | 01-01-2014 |
| | | ES 2733935 T3 | 03-12-2019 |
| | | PL 2678035 T3 | 30-09-2019 |
| | | TR 201909982 T4 | 21-08-2019 |
| | | US 2014039184 A1 | 06-02-2014 |
| | | WO 2012113860 A2 | 30-08-2012 |
| US 2020087259 A1 | 19-03-2020 | AU 2018234998 A1 | 07-11-2019 |
| | | BR 112019019324 A2 | 14-04-2020 |
| | | CA 3056486 A1 | 20-09-2018 |
| | | CN 110494421 A | 22-11-2019 |
| | | EP 3375774 A1 | 19-09-2018 |
| | | JP 2020514523 A | 21-05-2020 |
| | | KR 20190139873 A | 18-12-2019 |
| | | RU 2019132844 A | 19-04-2021 |
| | | SG 11201908281V A | 30-10-2019 |
| | | US 2020087259 A1 | 19-03-2020 |
| | | US 2022064114 A1 | 03-03-2022 |
| | | WO 2018167264 A1 | 20-09-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012113860 A
- WO 2018167264 A


### Non-patent literature cited in the description


- **CARS, O. ; CHANDY, S. J. ; MPUNDU, M. ; PERALTA, A. Q. ; ZORZET, A. ; SO, A. D.** Resetting the Agenda for Antibiotic Resistance through a Health Systems Perspective. *Lancet Glob. Health,* 2021, vol. 9, e1022-e1027
- **LEVY, S. B. ; MARSHALL, B.** Antibacterial Resistance Worldwide: Causes, Challenges and Responses. *Nat. Med.,* 2004, vol. 10, S122-S129
- UN Interagency Coordination Group on Antimicrobial Resistance. No time to wait: securing the future from drug-resistant infections. World Health Organization, 2019
- **MCMANUS, M. C.** Mechanisms of Bacterial Resistance to Antimicrobial Agents. *Am. J. Health. Syst. Pharm.,* 1997, vol. 54, 1420-1433
- **LLOR, C. ; BJERRUM, L.** Antimicrobial Resistance: Risk Associated with Antibiotic Overuse and Initiatives to Reduce the Problem. *Ther. Adv. Drug Saf,* 2014, vol. 5, 229-241
- **JIA, Q. ; SONG, Q. ; LI, P. ; HUANG, W.** Rejuvenated Photodynamic Therapy for Bacterial Infections. *Adv. Healthc. Mater,* 2019, vol. 8, 1900608
- **CIEPLIK, F. ; DENG, D. ; CRIELAARD, W. ; BUCHALLA, W. ; HELLWIG, E. ; AL-AHMAD, A. ; MAISCH, T.** Antimicrobial Photodynamic Therapy - What We Know and What We Don't. *Crit. Rev. Microbiol,* 2018, vol. 44, 571-589
- **HAMBLIN, M. R. ; HASAN, T.** Photodynamic Therapy: A New Antimicrobial Approach to Infectious Disease? Photochem. *Photobiol. Sci.,* 2004, vol. 3, 436-450
- Singlet Oxygen: Applications in Biosciences and Nanosciences. Comprehensive Series in Photochemical & Photobiological Sciences. The Royal Society of Chemistry, 2016
- **QUINA, F. H. ; SILVA, G. T. M.** The Photophysics of Photosensitization: A Brief Overview. *J. Photochem. Photobiol.,* 2021, vol. 7, 100042
- **SASIKUMAR, D. ; JOHN, A. T. ; SUNNY, J. ; HARIHARAN, M.** Access to the Triplet Excited States of Organic Chromophores. *Chem Soc Rev,* 2020, vol. 49, 6122-6140
- **BAPTISTA, M. S. ; CADET, J. ; DI MASCIO, P. ; GHOGARE, A. A. ; GREER, A. ; HAMBLIN, M. R. ; LORENTE, C. ; NUNEZ, S. C. ; RIBEIRO, M. S. ; THOMAS, A. H.** Type I and Type II Photosensitized Oxidation Reactions: Guidelines and Mechanistic Pathways. *Photochem. Photobiol.,* 2017, vol. 93, 912-919
- **ALMEIDA, A. ; FAUSTINO, M. A. ; TOMÉ, J. P.** Photodynamic Inactivation of Bacteria: Finding the Effective Targets. *Future Med. Chem.,* 2015, vol. 7, 1221-1224
- **ALVES, E. ; FAUSTINO, M. A. ; NEVES, M. G. ; CUNHA, A. ; TOME, J. ; ALMEIDA, A.** An Insight on Bacterial Cellular Targets of Photodynamic Inactivation. *Future Med. Chem.,* 2014, vol. 6, 141-164
- **MARASINI, S. ; LEANSE, L. G. ; DAI, T.** Can Microorganisms Develop Resistance against Light Based Anti-Infective Agents?. *Adv. Drug Deliv. Rev.,* 2021, vol. 175, 113822
- **PHAM, T. C. ; NGUYEN, V.-N. ; CHOI, Y. ; LEE, S. ; YOON, J.** Recent Strategies to Develop Innovative Photosensitizers for Enhanced Photodynamic Therapy. *Chem. Rev.,* 2021, vol. 121, 13454-13619
- **MAISCH, T.** Photoantimicrobials-An Update. *Transl. Biophotonics,* 2020, vol. 2
- **SCHMIDT, R. ; TANIELIAN, C. ; DUNSBACH, R. ; WOLFF, C.** Phenalenone, a Universal Reference Compound for the Determination of Quantum Yields of Singlet Oxygen O2(1Δg) Sensitization. *J. Photochem. Photobiol. Chem.,* 1994, vol. 79, 11-17
- **MARTÍ, C. ; JÜRGENS, O. ; CUENCA, O. ; CASALS, M. ; NONELL, S.** Aromatic Ketones as Standards for Singlet Molecular Oxygen O2(1Δg) Photosensitization. Time-Resolved Photoacoustic and near-IR Emission Studies. *J. Photochem. Photobiol. Chem.,* 1996, vol. 97, 11-18
- **OLIVEROS, E. ; H. BOSSMANN, S. ; NONELL, S. ; MARTÍ, C. ; HEIT, G. ; TRÖSCHER, G. ; NEUNER, A. ; MARTINEZ, C. ; M. BRAUN.** A. Photochemistry of the Singlet Oxygen [O2(1Δg)] Sensitizer Perinaphthenone (Phenalenone) in N,N'-Dimethylacetamide and 1,4-Dioxane. *New J Chem,* 1999, vol. 23, 85-93

- **FLORS, C. ; NONELL, S.** Light and Singlet Oxygen in Plant Defense Against Pathogens: Phototoxic Phenalenone Phytoalexins. *Acc. Chem. Res,* 2006, vol. 39, 293-300
- **GODARD, J. ; GIBBONS, D. ; LEROY-LHEZ, S. ; WILLIAMS, R. M. ; VILLANDIER, N. ; OUK, T.-S. ; ; BRÉGIER, F. ; SOL, V.** Development of Phenalenone-Triazolium Salt Derivatives for APDT: Synthesis and Antibacterial Screening. *Antibiotics,* 2021, vol. 10, 626
- **MUEHLER, D. ; RUPP, C. M. ; KECELI, S. ; BROCHHAUSEN, C. ; SIEGMUND, H. ; MAISCH, T. ; HILLER, K.-A. ; BUCHALLA, W. ; CIEPLIK, F.** Insights Into Mechanisms of Antimicrobial Photodynamic Action Toward Biofilms Using Phenalen-1-One Derivatives as Photosensitizers. *Front. Microbiol.,* 2020, vol. 11, 2594
- **CIEPLIK, F. ; WIMMER, F. ; MUEHLER, D. ; THURNHEER, T. ; BELIBASAKIS, G. N. ; HILLER, K.-A. ; MAISCH, T. ; BUCHALLA, W.** Phenalen-1-One-Mediated Antimicrobial Photodynamic Therapy and Chlorhexidine Applied to a Novel Caries Biofilm Model. *Caries Res.,* 2018, vol. 52, 447-453
- **MUEHLER, D. ; SOMMER, K. ; WENNIGE, S. ; HILLER, K.-A. ; CIEPLIK, F. ; MAISCH, T. ; SPÄTH, A.** Light-Activated Phenalen-1-One Bactericides: Efficacy, Toxicity and Mechanism Compared with Benzalkonium Chloride. *Future Microbiol.,* 2017, vol. 12, 1297-1310
- **TABENSKI, I. ; CIEPLIK, F. ; TABENSKI, L. ; REGENSBURGER, J. ; HILLER, K.-A. ; BUCHALLA, W. ; MAISCH, T. ; SPÄTH, A.** The Impact of Cationic Substituents in Phenalen-1-One Photosensitizers on Antimicrobial Photodynamic Efficacy. *Photochem. Photobiol. Sci.,* 2016, vol. 15, 57-68
- **SPÄTH, A. ; LEIBL, C. ; CIEPLIK, F. ; LEHNER, K. ; REGENSBURGER, J. ; HILLER, K.-A. ; BÄUMLER, W. ; SCHMALZ, G. ; MAISCH, T.** Improving Photodynamic Inactivation of Bacteria in Dentistry: Highly Effective and Fast Killing of Oral Key Pathogens with Novel Tooth-Colored Type-II Photosensitizers. *J. Med. Chem.,* 2014, vol. 57, 5157-5168
- **CIEPLIK, F. ; SPÄTH, A. ; REGENSBURGER, J. ; GOLLMER, A. ; TABENSKI, L. ; HILLER, K.-A. ; BÄUMLER, W. ; MAISCH, T. ; SCHMALZ, G.** Photodynamic Biofilm Inactivation by SAPYR-An Exclusive Singlet Oxygen Photosensitizer. *Free Radic. Biol. Med.,* 2013, vol. 65, 477-487
- **BRESOLÍ-OBACH, R. ; GISPERT, I. ; PEÑA, D. G. ; BOGA, S. ; GULIAS, Ó. ; AGUT, M. ; VÁZQUEZ, M. E. ; NONELL, S.** Triphenylphosphonium Cation: A Valuable Functional Group for Antimicrobial Photodynamic Therapy. *J. Biophotonics,* 2018, vol. 11
- **CARDEN, R. G. ; SOMMERS, K. J. ; SCHRANK, C. L. ; LEITGEB, A. J. ; FELICIANO, J. A. ; WUEST, W. M. ; MINBIOLE, K. P. C.** Advancements in the Development of Non-Nitrogen-Based Amphiphilic Antiseptics to Overcome Pathogenic Bacterial Resistance. *ChemMedChem,* 2020, vol. 15, 1974-1984
- **DE BONFILS, P. ; VERRON, E. ; SANDOVAL-ALTAMIRANO, C. ; JAQUE, P. ; MOREAU, X. ; GUNTHER, G. ; NUN, P. ; COEFFARD, V.** Unusual Oxidative Dealkylation Strategy toward Functionalized Phenalenones as Singlet Oxygen Photosensitizers and Photophysical Studies. *J. Org. Chem.,* 2020, vol. 85, 10603-10616
- **KAYE, E. G. ; KAILASS, K. ; SADOVSKI, O. ; BEHARRY, A. A.** A Green-Absorbing, Red-Fluorescent Phenalenone-Based Photosensitizer as a Theranostic Agent for Photodynamic Therapy. *ACS Med. Chem. Lett.,* 2021, vol. 12, 1295-1301
- **KHARKWAL, G. B. ; SHARMA, S. K. ; HUANG, Y.-Y. ; DAI, T. ; HAMBLIN, M. R.** Photodynamic Therapy for Infections: Clinical Applications. *Lasers Surg. Med.,* 2011, vol. 43, 755-767
- **LIN, G. ; HU, M. ; ZHANG, R. ; ZHU, Y. ; GU, K. ; BAI, J. ; LI, J. ; DONG, X. ; ZHAO, W.** Discovery of Meso-(Meta-Pyridinium) BODIPY Photosensitizers: In Vitro and In Vivo Evaluations for Antimicrobial Photodynamic Therapy. *J. Med. Chem.,* 2021, vol. 64, 18143-18157
- **SANDOVAL-ALTAMIRANO, C. ; DE LA FUENTE, J. R. ; BERRIOS, E. ; SANCHEZ, S. A. ; PIZARRO, N. ; MORALES, J. ; GUNTHER, G.** Photophysical Characterization of Hydroxy and Ethoxy Phenalenone Derivatives. *J. Photochem. Photobiol. Chem.,* 2018, vol. 353, 349-357
- **LAVIS, L. D. ; RAINES, R. T.** Bright Ideas for Chemical Biology. *ACS Chem. Biol.,* 2008, vol. 3 (3), 142-155
- **DAZA, M. C. ; DOERR, M. ; SALZMANN, S. ; MARIAN, C. M. ; THIEL, W.** Photophysics of Phenalenone: Quantum-Mechanical Investigation of Singlet-Triplet Intersystem Crossing. *Phys Chem Chem Phys,* 2009, vol. 11, 1688-1696
- **BREGNHØJ, M. ; WESTBERG, M. ; JENSEN, F. ; OGILBY, P. R.** Solvent-Dependent Singlet Oxygen Lifetimes: Temperature Effects Implicate Tunneling and Charge-Transfer Interactions. *Phys Chem Chem Phys,* 2016, vol. 18, 22946-22961
- **TURRO, N. J. ; RAMAMURTHY, V. ; SCAIANO, J. C.** Modern Molecular Photochemistry of Organic Molecules. University Science Books, 2010
- **KUZNETSOVA, N. A. ; GRETSOVA, N. S. ; YUZHAKOVA, O. A. ; NEGRIMOVSKII, V. M. ; KALIYA, O. L. ; LUK'YANETS, E. A.** New Reagents for Determination of the Quantum Efficiency of Singlet Oxygen Generation in Aqueous Media. *Russ. J. Gen. Chem.,* 2001, vol. 71, 36-41

- **RICE, L. B.** Federal Funding for the Study of Antimicrobial Resistance in Nosocomial Pathogens: No ESKAPE. *J. Infect. Dis.,* 2008, vol. 197, 1079-1081
- **DE BONFILS, P. ; VERRON, E. ; NUN, P. ; COEFFARD, V.** Photoinduced Storage and Thermal Release of Singlet Oxygen from 1,2-Dihydropyridine Endoperoxides. *ChemPhotoChem,* 2021, vol. 5, 847-856